(19) Europäisches Patentamt
European Patent Office
Office européen des brevets

(11) EP 4 177 276 A1

(12) **DEMANDE DE BREVET EUROPEEN**

(43) Date de publication:
**10.05.2023 Bulletin 2023/19**

(21) Numéro de dépôt: **21206821.7**

(22) Date de dépôt: **06.11.2021**

(51) Classification Internationale des Brevets (IPC):
***C08B 37/08*** *(2006.01)* ***C08L 5/08*** *(2006.01)*
***A61K 31/728*** *(2006.01)* ***A61K 47/36*** *(2006.01)*

(52) Classification Coopérative des Brevets (CPC):
**C08B 37/0072; A61K 31/00; A61K 47/36; C08L 5/08**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(71) Demandeur: **Laboratoires Vivacy**
**75116 Paris (FR)**

(72) Inventeurs:
- **TRANCHEPAIN, Frédéric**
**74350 Villy Le Bouveret (FR)**
- **BRUNEL, Florence**
**74100 Annemasse (FR)**

(74) Mandataire: **Tripoz, Inès**
**Cabinet Tripoz**
**Le Pôle Sud**
**22 rue Seguin**
**69002 Lyon (FR)**

(54) **COMPOSITION, SOUS FORME DE SOLUTION AQUEUSE COMPRENANT AU MOINS UN COMPOSE MACROMOLECULAIRE**

(57) La présente invention concerne une composition, sous forme de solution aqueuse, filtrable sur membrane de porosité 0,22 μm comprenant au moins un composé macromoléculaire constitué d'enchaînements de polysaccharides identiques ou différents liés entre eux par un radical divalent L.

L est un radical divalent issu d'une réaction entre un agent réticulant et deux fonctions réactives, chaque fonction réactive étant portée par deux chaines distinctes de polysaccharide et les enchaînements de polysaccharides ne forment pas de structures cycliques.

EP 4 177 276 A1

## Description

L'invention concerne le domaine des formulations à base de polysaccharide utilisées en tant que biomatériaux, plus particulièrement dans les domaines médicaux et esthétiques. Dans ces applications, les formulations doivent présenter des propriétés optimisées en termes de rhéologie tenant compte notamment des caractéristiques du dispositif médical utilisé pour l'injection, des caractéristiques de la zone traitée et de l'effet thérapeutique ou cosmétique recherché.

Les produits sous forme de gel à base de polysaccharide réticulé utilisés pour une application esthétique ont des propriétés rhéologiques optimisées pour avoir une bonne tenue dans la zone d'injection. Les gels utilisés présentent notamment une fermeté à la déformation qui se traduit par une tangente de perte, Tan $\Delta$ (Tn $\delta$), inférieure à 1, le module d'élasticité G' étant bien supérieur au module de viscosité G" sur une large plage de déformation. En particulier, la plupart des produits utilisés sous forme de gel présentent une tangente de perte inférieure à 0,5 (REF). Cette caractéristique des produits à base de gel impose une certaine contrainte en terme d'injectabilité, ce qui peut limiter leur domaine d'utilisation. En particulier, le recours à un tel produit pour des zones sensibles du corps ou du visage peut être délicat, voir problématique.

La présente invention concerne la mise au point de compositions sous forme de solutions aqueuses, présentant une très bonne fluidité, filtrables sur membrane de porosité 0,22 $\mu$m, ces compositions comprenant au moins un composé macromoléculaire linéaire ou branché constitué d'un enchaînement de polysaccharides.

De façon surprenante, les compositions selon l'invention présentent l'intérêt remarquable d'être filtrables sur une membrane de porosité 0,22 $\mu$m, ce qui permet d'incorporer dans celles-ci des composés thermosensibles tels que des peptides, protéines, facteurs de croissance, anticorps ou des vitamines notamment, car les compositions obtenues peuvent alors être stérilisées par simple filtration. Les produits existants à base de gels de polysaccharides réticulés sont incompatibles avec des composés thermosensibles car un traitement à l'autoclave ou une stérilisation à la chaleur du gel est nécessaire pour éviter tout risque d'infection sur la zone traitée par injection.

La présente invention concerne également des compositions comprenant au moins un composé macromoléculaire constitué d'un enchaînement d'acides hyaluroniques présentant, après injection, une bonne durabilité et résistance à la dégradation enzymatique, en particulier.

Les compositions selon la présente invention permettent également la préparation de solutions dont les concentrations en polysaccharide sont élevées, en acide hyaluronique notamment, tout en restant cependant facilement injectables.

La présente invention concerne une composition, sous forme de solution aqueuse, filtrable sur membrane de porosité 0,22 $\mu$m comprenant au moins un composé macromoléculaire constitué d'enchaînements de polysaccharides identiques ou différents liés entre eux par un radical divalent L.

L est un radical divalent issu d'une réaction entre un agent réticulant et deux fonctions réactives, chaque fonction réactive étant portée par deux chaines distinctes de polysaccharide et les enchaînements de polysaccharides ne forment pas de structures cycliques.

Dans un mode de réalisation, la composition selon l'invention est caractérisée en ce que le composé macromoléculaire est constitué d'un enchaînement de polysaccharides choisis dans le groupe constitué de l'acide hyaluronique, du kératane, de l'héparine, de la cellulose, des dérivés de cellulose, de l'acide alginique, du xanthane, du carraghénane, du chitosan, de la chondroïtine, de l'héparosan, et leurs sels biologiquement acceptables, seul(s) ou en mélange.

Dans un mode de réalisation, la composition selon l'invention est caractérisée en ce que le composé macromoléculaire est constitué d'un enchaînement d'acides hyaluroniques.

Dans un mode de réalisation, la composition selon l'invention est caractérisée en ce que le composé macromoléculaire est constitué d'un enchaînement d'héparosan.

Dans un mode de réalisation, la composition selon l'invention est caractérisée en ce que le composé macromoléculaire est constitué d'un enchaînement de polysaccharides choisis dans le groupe constitué d'acide hyaluronique, d'héparosan ou de leurs sels respectifs.

L'invention concerne également un procédé de préparation d'une composition comprenant au moins un composé macromoléculaire constitué d'un enchaînement de polysaccharides, d'acide hyaluronique, par exemple, et plus particulièrement un procédé de préparation permettant l'obtention d'une composition comprenant au moins un composé macromoléculaire constitué d'un enchaînement de polysaccharides présentant des propriétés particulières comme la filtrabilité sur membrane de porosité 0,22 $\mu$m.

Le procédé de préparation des compositions selon l'invention comprend au moins les étapes suivantes :

a) on dispose d'un polysaccharide ;
b) on dispose d'un agent réticulant ;
c) on met en œuvre une ou plusieurs étape(s) de réticulation en présence dudit polysaccharide et dudit agent réticulant ;
d) on obtient un polysaccharide réticulé ;

e) on met en œuvre une ou plusieurs étape(s) de rupture de liaisons glycosidiques ;
f) on obtient une solution de composé macromoléculaire ;
g) on filtre la solution de composé macromoléculaire sur membrane de porosité 0,22 µm.

Dans un mode de réalisation, le procédé de préparation des compositions selon l'invention comprend au moins les étapes suivantes :

a) on dispose d'un polysaccharide ;
b) on dispose d'un agent réticulant ;
c) on met en œuvre une ou plusieurs étape(s) de réticulation en présence dudit polysaccharide et dudit agent réticulant ;
d) on obtient un polysaccharide réticulé ;
e) on met en œuvre une étape de rupture de liaisons glycosidiques ;
f) on obtient une solution de composé macromoléculaire ;
g) on filtre sur membrane de porosité 0,22 µm un échantillon de la solution de composé macromoléculaire. Il s'agit d'un test de contrôle en cours du procédé ou in-process control (IPC).

Dans un mode de réalisation, le procédé de préparation des compositions selon l'invention comprend au moins les étapes suivantes :

a) on dispose d'un polysaccharide ;
b) on dispose d'un agent réticulant ;
c) on met en œuvre une ou plusieurs étape(s) de réticulation en présence dudit polysaccharide et dudit agent réticulant à partir d'une solution d'au moins un polysaccharide à une concentration supérieure ou égale à 10% en masse, par rapport à la masse totale du milieu réactionnel de réticulation et en travaillant à température constante ou à température variable de façon linéaire ou par paliers, ladite température constante ou variable étant inférieure ou égale à 10 °C pour un temps de réaction d'au plus 24 heures ;
d) on obtient un polysaccharide réticulé ;
e) on met en œuvre une étape de rupture de liaisons glycosidiques ;
f) on obtient une solution de composé macromoléculaire ;
g) on filtre sur membrane de porosité 0,22 µm la solution de composé macromoléculaire.

Dans un mode de réalisation, le procédé de préparation des compositions selon l'invention comprend au moins les étapes suivantes :

a) on dispose d'un polysaccharide ;
b) on dispose d'un agent réticulant ;
c) on met en œuvre une ou plusieurs étape(s) de réticulation en présence dudit polysaccharide et dudit agent réticulant à partir d'une solution d'au moins un polysaccharide à une concentration supérieure ou égale à 10% en masse, par rapport à la masse totale du milieu réactionnel de réticulation et en travaillant à température constante ou à température variable de façon linéaire ou par paliers, ladite température constante ou variable étant inférieure ou égale à 10 °C pour un temps de réaction d'au plus 24 heures ;
d) on obtient un polysaccharide réticulé ;
e) on met en œuvre une étape de rupture de liaisons glycosidiques ;
f) on obtient une solution de composé macromoléculaire ;
g) on filtre sur membrane de porosité 0,22 µm un échantillon de la solution de composé macromoléculaire. Il s'agit d'un test de contrôle en cours du procédé ou in-process control (IPC).

Lors de l'étape d) on obtient un réseau constitué de polysaccharides différents liés entre eux par un radical divalent L, issu de l'agent réticulant.
Dans un mode de réalisation, le procédé selon l'invention est caractérisé en ce que l'agent réticulant est choisi dans le groupe constitué par les bis-époxydes, les trimétaphosphates, les diamines, les dialcoxyamines et les dihydrazides.
Dans un mode de réalisation, le procédé selon l'invention est caractérisé en ce que l'agent réticulant est le 1,4-butanediol diglycidyl éther (BDDE).
Dans un mode de réalisation, le procédé selon l'invention est caractérisé en ce que l'agent réticulant est un sel de trimétaphosphate.
Dans un mode de réalisation, le procédé selon l'invention est caractérisé en ce que l'agent réticulant est une

diamine.

Dans un mode de réalisation, le procédé selon l'invention est caractérisé en ce que l'agent réticulant est une dihydrazide.

Dans un mode de réalisation, l'étape e) de rupture de liaisons glycosidiques mise en œuvre sur au moins un polysaccharide réticulé conduit à la destruction majoritaire du réseau de réticulation formé lors de l'étape c).

Dans un mode de réalisation, l'étape e) de rupture de liaisons glycosidiques mise en œuvre sur au moins un polysaccharide réticulé conduit à la destruction complète du réseau de réticulation formé lors de l'étape c).

Los de cette étape e), seules des liaisons glycosidiques sont rompues à l'exception des liaisons entre le radical divalent L, issu de l'agent réticulant et le polysaccharide.

Dans un mode de réalisation, le procédé selon l'invention est caractérisé en ce que l'étape e) de rupture de liaisons glycosidiques est effectuée au moyen d'un traitement chimique.

Dans un mode de réalisation, le procédé selon l'invention est caractérisé en ce que l'étape e) de rupture de liaisons glycosidiques est effectuée au moyen d'un traitement thermique.

Dans un mode de réalisation, le procédé selon l'invention est caractérisé en ce que le traitement thermique de l'étape e) de rupture de liaisons glycosidiques est effectué par autoclavage à la vapeur.

Dans un mode de réalisation, le procédé selon l'invention est caractérisé en ce que l'étape e) de rupture de liaisons glycosidiques est effectuée au moyen d'un traitement par radiations.

Dans un mode de réalisation, le procédé selon l'invention est caractérisé en ce que l'étape e) de rupture de liaisons glycosidiques est effectuée au moyen d'un traitement enzymatique.

Dans un mode de réalisation, le procédé selon l'invention est caractérisé en ce que l'étape e) de rupture de liaisons glycosidiques est effectuée au moyen d'un traitement à haute pression.

L'invention concerne également un composé macromoléculaire de formule générale I :

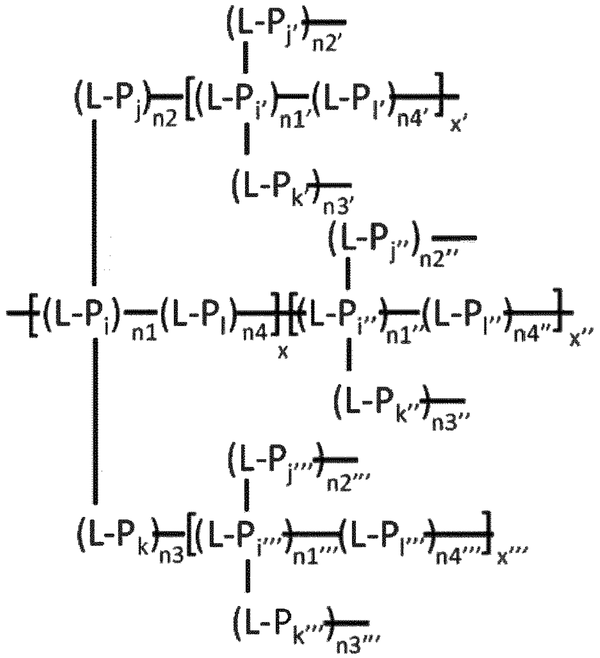

Formule I

dans laquelle :

- $P_i$, $P_{i'}$, $P_{i''}$, $P_{i'''}$, $P_j$, $P_{j'}$, $P_{j''}$, $P_{j'''}$, $P_k$, $P_{k'}$, $P_{k''}$, $P_{k'''}$, $P_l$, $P_{l'}$, $P_{l''}$ et $P_{l'''}$ sont des polysaccharides identiques ou différents
- n1, n1', n1", n1‴, n2, n2', n2", n2‴, n3, n3', n3", n3‴, n4, n4', n4" et n4‴ sont des entiers compris entre 0 et 2000 et au moins un des n1, n1', n1", n1‴, n2, n2', n2", n2‴, n3, n3', n3", n3‴, n4, n4', n4" et n4‴ est supérieur ou égal à 2
- x, x', x" et x‴ sont des entiers supérieurs ou égaux à 0
- L est un radical divalent issu d'une réaction entre un agent réticulant et deux fonctions réactives, chaque fonction réactive étant portée par deux chaines distinctes de polysaccharide
- les enchaînements de polysaccharides ne forment pas de structures cycliques

Dans un mode de réalisation, n1, n1', n1", n1‴, n2, n2', n2", n2‴, n3, n3', n3", n3‴, n4, n4', n4" et n4‴ sont des entiers compris entre 0 et 1500 et au moins un des n1, n1', n1", n1‴, n2, n2', n2", n2‴, n3, n3', n3", n3‴, n4, n4', n4" et

n4‴ est supérieur ou égal à 2.

Dans un mode de réalisation, n1, n1’, n1", n1‴, n2, n2’, n2", n2‴, n3, n3’, n3", n3‴, n4, n4’, n4" et n4‴ sont des entiers compris entre 0 et 1000 et au moins un des n1, n1’, n1", n1‴, n2, n2’, n2", n2‴, n3, n3’, n3", n3‴, n4, n4’, n4" et n4‴ est supérieur ou égal à 2.

Dans un mode de réalisation, n1, n1’, n1", n1‴, n2, n2’, n2", n2‴, n3, n3’, n3", n3‴, n4, n4’, n4" et n4‴ sont des entiers compris entre 0 et 500 et au moins un des n1, n1’, n1", n1‴, n2, n2’, n2", n2‴, n3, n3’, n3", n3‴, n4, n4’, n4" et n4'" est supérieur ou égal à 2.

Dans un mode de réalisation, n1, n1’, n1", n1‴, n2, n2’, n2", n2‴, n3, n3’, n3", n3‴, n4, n4’, n4" et n4‴ sont des entiers compris entre 0 et 250 et au moins un des n1, n1’, n1", n1‴, n2, n2’, n2", n2‴, n3, n3’, n3", n3‴, n4, n4’, n4" et n4‴ est supérieur ou égal à 2.

Dans un mode de réalisation, n1, n1’, n1", n1‴, n2, n2’, n2", n2‴, n3, n3’, n3", n3‴, n4, n4’, n4" et n4‴ sont des entiers compris entre 0 et 150 et au moins un des n1, n1’, n1", n1‴, n2, n2’, n2", n2‴, n3, n3’, n3", n3‴, n4, n4’, n4" et n4‴ est supérieur ou égal à 2.

Dans un mode de réalisation, n1, n1’, n1", n1‴, n2, n2’, n2", n2‴, n3, n3’, n3", n3‴, n4, n4’, n4" et n4‴ sont des entiers compris entre 0 et 100 et au moins un des n1, n1’, n1", n1‴, n2, n2’, n2", n2‴, n3, n3’, n3", n3‴, n4, n4’, n4" et n4‴ est supérieur ou égal à 2.

Dans un mode de réalisation, n1, n1’, n1", n1‴, n2, n2’, n2", n2‴, n3, n3’, n3", n3‴, n4, n4’, n4" et n4‴ sont des entiers compris entre 0 et 50 et au moins un des n1, n1’, n1", n1‴, n2, n2’, n2", n2‴, n3, n3’, n3", n3‴, n4, n4’, n4" et n4‴ est supérieur ou égal à 2.

Dans un mode de réalisation, n1, n1’, n1", n1‴, n2, n2’, n2", n2‴, n3, n3’, n3", n3‴, n4, n4’, n4" et n4‴ sont des entiers compris entre 0 et 30 et au moins un des n1, n1’, n1", n1‴, n2, n2’, n2", n2‴, n3, n3’, n3", n3‴, n4, n4’, n4" et n4'" est supérieur ou égal à 2.

L’invention concerne une composition sous forme de solution aqueuse, filtrable sur membrane de porosité 0,22 μm comprenant au moins un composé macromoléculaire constitué d’enchaînements de polysaccharides identiques ou différents liés entre eux par un radical divalent L, caractérisée en ce que le composé macromoléculaire a pour formule générale I :

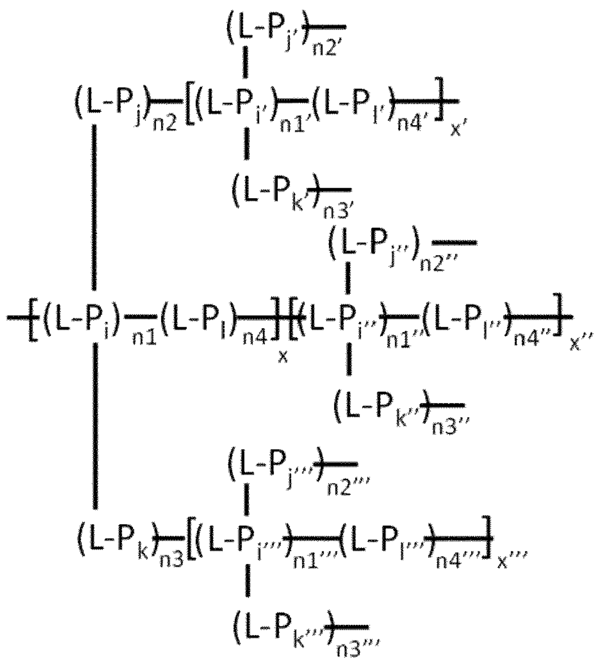

Formule I

dans laquelle :

- $P_i$, $P_{i'}$, $P_{i''}$, $P_{i'''}$, $P_j$, $P_{j'}$, $P_{j''}$, $P_{j'''}$, $P_k$, $P_{k'}$, $P_{k''}$, $P_{k'''}$, $P_l$, $P_{l'}$, $P_{l''}$ et $P_{l'''}$ sont des polysaccharides identiques ou différents
- n1, n1’, n1", n1‴, n2, n2’, n2", n2‴, n3, n3’, n3", n3"’, n4, n4’, n4" et n4‴ sont des entiers compris entre 0 et 2000 et au moins un des n1, n1’, ni", n1‴, n2, n2’, n2", n2‴, n3, n3’, n3", n3‴, n4, n4’, n4" et n4‴ est supérieur ou égal à 2
- x, x’, x" et x'" sont des entiers supérieurs ou égaux à 0
- L est un radical divalent issu d’une réaction entre un agent réticulant et deux fonctions réactives, chaque fonction réactive étant portée par deux chaines distinctes de polysaccharide

- les enchaînements de polysaccharides ne forment pas de structures cycliques.

Dans un mode de réalisation, n1, n1', n1", n1‴, n2, n2', n2", n2‴, n3, n3', n3", n3‴, n4, n4', n4" et n4‴ sont des entiers compris entre 0 et 1500 et au moins un des n1, n1', n1", n1‴, n2, n2', n2", n2‴, n3, n3', n3", n3‴, n4, n4', n4" et n4‴ est supérieur ou égal à 2.

Lorsque le composé macromoléculaire selon l'invention sous forme sèche est hydraté au moyen d'une solution aqueuse, une solution aqueuse du composé macromoléculaire est obtenue.

En particulier, le composé macromoléculaire selon l'invention ne présente pas de capacité de gonflement.

Dans le sens de l'invention, on entend par capacité de gonflement le fait qu'un composé sous forme sèche mis en présence d'une solution aqueuse, une solution saline par exemple, a la capacité à former un gel de volume remarquable dont plus de 80% est retenu par filtration sur membrane de porosité 0,22 $\mu$m. La formation d'un gel est caractérisée par la présence de deux phases dans le milieu : le gel ou « solide mou » et la solution ou surnageant.

En présence d'eau, le composé macromoléculaire selon l'invention ne forme pas de gel.

En particulier, le composé macromoléculaire selon l'invention ne forme pas un hydrogel. Dans le cadre de la présente demande, on appelle « hydrogel » un gel constitué d'un réseau tridimensionnel constitué d'au moins un composé, susceptible d'absorber une grande quantité d'eau ou de solution aqueuse et qui présente des propriétés rhéologiques particulières notamment en termes de viscosité et de viscoélasticité.

Dans un mode de réalisation, ledit polysaccharide de l'étape a) est choisi dans le groupe des glycosaminoglycanes (GAG).

Dans un mode de réalisation, ledit polysaccharide de l'étape a) est choisi dans le groupe des polysaccharides modifiés chimiquement, oxydés ou substitués.

Dans un mode de réalisation, ledit polysaccharide est choisi dans le groupe des glycosaminoglycanes (GAG), comme par exemples la chondroïtine, le kératane, l'héparine, l'héparosan ou encore l'acide hyaluronique, et leurs mélanges.

Dans un mode de réalisation, ledit polysaccharide est choisi dans le groupe constitué de l'acide hyaluronique, du kératane, de l'héparine, de la cellulose, des dérivés de cellulose, de la cellulose oxydée, de l'acide alginique, du xanthane, du carraghénane, du chitosane, de la chondroïtine, de l'héparosan et leur sels biologiquement acceptables, seuls ou en mélange.

Dans un mode de réalisation, ledit polysaccharide est l'acide hyaluronique, ou l'un de ses sels biologiquement acceptables, seuls ou en mélange.

Dans le cadre de la présente demande, l'acide hyaluronique, ou l'un de ses sels biologiquement acceptables, seuls ou en mélange, sont préférés.

Dans un mode de réalisation, ledit polysaccharide est choisi dans le groupe constitué de l'acide hyaluronique, du hyaluronate de sodium, et de leurs mélanges.

Dans un mode de réalisation, ledit polysaccharide est l'acide hyaluronique.

Dans un mode de réalisation, ledit polysaccharide est choisi dans le groupe constitué du hyaluronate de sodium et du hyaluronate de potassium.

Dans un mode de réalisation, ledit polysaccharide est le hyaluronate de sodium.

Dans le cadre de la présente demande, le hyaluronate de sodium est le polysaccharide particulièrement préféré.

Dans un mode de réalisation, ledit polysaccharide est un acide hyaluronique, ou l'un de ses sels, modifié chimiquement par substitution.

Dans un mode de réalisation, ledit polysaccharide est un acide hyaluronique, ou l'un de ses sels, substitué par un groupement apportant des propriétés lipophile ou hydratante, comme par exemple les acides hyaluroniques substitués tels que décrits dans la demande de brevet FR 2 983 483 au nom de la demanderesse.

Dans un mode de réalisation, ledit polysaccharide est l'héparosan, ou l'un de ses sels biologiquement acceptables, seuls ou en mélange.

Dans le cadre de la présente demande, l'héparosan, ou l'un de ses sels biologiquement acceptables, seuls ou en mélange, sont préférés.

Dans un mode de réalisation, ledit polysaccharide est choisi dans le groupe constitué de l'héparosan, de l'héparosan de sodium, et de leurs mélanges.

Dans un mode de réalisation, ledit polysaccharide est l'héparosan.

Dans un mode de réalisation, ledit polysaccharide est choisi dans le groupe constitué de l'héparosan de sodium et de l'héparosan de potassium.

Dans un mode de réalisation, ledit polysaccharide est l'héparosan de sodium.

Dans le cadre de la présente demande, l'héparosan de sodium est le polysaccharide particulièrement préféré.

Dans un mode de réalisation, ledit polysaccharide est un héparosan, ou l'un de ses sels, modifié chimiquement.

Dans un mode de réalisation, ledit polysaccharide est choisi dans le groupe constitué de la cellulose et des dérivés de cellulose.

Dans un mode de réalisation, ledit polysaccharide est choisi dans le groupe constitué des dérivés de cellulose comprenant notamment l'hydroxypropylcellulose, l'hydroxypropylméthylcellulose, l'éthylméthyle cellulose et la carboxyméthylcellulose.

Dans un mode de réalisation, ledit polysaccharide est la cellulose.

Dans un mode de réalisation, ledit polysaccharide est un dérivé de cellulose.

Dans un mode de réalisation, ledit polysaccharide de l'étape a) du procédé de préparation des compositions selon l'invention est un mélange de polysaccharides.

Dans le cadre de la présente demande, tous les polysaccharides cités peuvent être mis en présence sous forme de mélange lors de l'étape a), qu'il s'agisse de polysaccharide de même nature (par exemple un mélange d'acide hyaluronique ayant des masses moléculaires différentes) ou de nature différente (par exemple un mélange d'acide hyaluronique et de chitosan). Lors de l'étape de réticulation, il pourra y avoir co-réticulation entre les différents polysaccharides.

Dans un mode de réalisation, le procédé de préparation des compositions selon l'invention est caractérisé en ce que ledit polysaccharide de l'étape a) est un mélange d'acides hyaluroniques, ou de sels d'acides hyaluroniques.

Dans un mode de réalisation, le procédé de préparation des compositions selon l'invention est caractérisé en ce que ledit polysaccharide de l'étape a) est un mélange de 2 acides hyaluroniques ou sels d'acides hyaluroniques.

Dans un mode de réalisation, le procédé de préparation des compositions selon l'invention est caractérisé en ce que ledit polysaccharide de l'étape a) est un mélange de 3 acides hyaluroniques ou sels d'acides hyaluroniques.

Dans un mode de réalisation, le procédé de préparation des compositions selon l'invention est caractérisé en ce que ledit polysaccharide de l'étape a) est un mélange de 4 acides hyaluroniques ou sels d'acides hyaluroniques.

Dans un mode de réalisation, le procédé de préparation des compositions selon l'invention est caractérisé en ce que ledit polysaccharide de l'étape a) est un mélange d'héparosans, ou de sels d'héparosans.

Dans un mode de réalisation, le procédé de préparation des compositions selon l'invention est caractérisé en ce que ledit polysaccharide de l'étape a) est un mélange de 2 héparosans ou sels d'héparosans.

Dans un mode de réalisation, le procédé de préparation des compositions selon l'invention est caractérisé en ce que ledit polysaccharide de l'étape a) est un mélange de 3 héparosans ou sels d'héparosans.

Dans un mode de réalisation, le procédé de préparation des compositions selon l'invention est caractérisé en ce que ledit polysaccharide de l'étape a) est un mélange de 4 héparosans ou sels d'héparosans.

Dans un mode de réalisation, le procédé de préparation des compositions selon l'invention est caractérisé en ce que ledit polysaccharide de l'étape a) est un mélange d'acides hyaluroniques et d'héparosans, ou de leurs sels.

Dans un mode de réalisation, le procédé de préparation des compositions selon l'invention est caractérisé en ce que ledit polysaccharide de l'étape a) est un mélange d'un acide hyaluronique et d'un héparosan, ou de leurs sels.

Dans un mode de réalisation, le procédé de préparation des compositions selon l'invention est caractérisé en ce que ledit polysaccharide de l'étape a) est un mélange de 2 acides hyaluroniques et d'un héparosan, ou de leurs sels.

Dans un mode de réalisation, le procédé de préparation des compositions selon l'invention est caractérisé en ce que ledit polysaccharide de l'étape a) est un mélange d'un acide hyaluronique et de 2 héparosans, ou de leurs sels.

Dans un mode de réalisation, le procédé de préparation des compositions selon l'invention est caractérisé en ce que ledit polysaccharide de l'étape a) est un mélange de 2 acides hyaluroniques et de 2 héparosans, ou de leurs sels.

Dans un mode de réalisation, le procédé de préparation des compositions selon l'invention est caractérisé en ce que ledit polysaccharide de l'étape a) est un mélange d'acides hyaluroniques ou de leurs sels et de celluloses.

Dans un mode de réalisation, le procédé de préparation des compositions selon l'invention est caractérisé en ce que ledit polysaccharide de l'étape a) est un mélange d'un acide hyaluronique ou de son sel et d'une cellulose.

Dans un mode de réalisation, le procédé de préparation des compositions selon l'invention est caractérisé en ce que ledit polysaccharide de l'étape a) est un mélange de 2 acides hyaluroniques ou de leurs sels et d'une cellulose.

Dans un mode de réalisation, le procédé de préparation des compositions selon l'invention est caractérisé en ce que ledit polysaccharide de l'étape a) est un mélange d'un acide hyaluronique ou de son sel et de 2 celluloses.

Dans un mode de réalisation, le procédé de préparation des compositions selon l'invention est caractérisé en ce que ledit polysaccharide de l'étape a) est un mélange de 2 acides hyaluroniques ou de leurs sels et de 2 celluloses.

Dans le cadre de la présente demande, on appelle Mw ou « masse moléculaire », la masse moléculaire moyenne en poids des polysaccharides, mesurée en Daltons.

Dans un mode de réalisation, ledit polysaccharide ou l'un de ses sels a une masse moléculaire de 0,01 MDa à 10 MDa ( 0,01 MDa $\leq$ Mw $\leq$ 10 MDa).

Dans un mode de réalisation, ledit polysaccharide ou l'un de ses sels a une masse moléculaire comprise entre 0,01 MDa et 8 MDa.

Dans un mode de réalisation, ledit polysaccharide ou l'un de ses sels a une masse moléculaire comprise entre 0,01 MDa et 5 MDa.

Dans un mode de réalisation, ledit polysaccharide ou l'un de ses sels a une masse moléculaire comprise entre 0,01 MDa et 3,5 MDa.

Dans un mode de réalisation, ledit polysaccharide ou l'un de ses sels a une masse moléculaire comprise entre 0,5 MDa et 3,5 MDa.

Dans un mode de réalisation, ledit polysaccharide ou l'un de ses sels a une masse moléculaire comprise entre 2,75 MDa et 3,25 MDa.

Dans un mode de réalisation, ledit polysaccharide ou l'un de ses sels a une masse moléculaire comprise entre 0,75 MDa et 1,25 MDa.

Dans un mode de réalisation, ledit polysaccharide ou l'un de ses sels a une masse moléculaire comprise entre 2 MDa et 5 MDa.

Dans un mode de réalisation, ledit polysaccharide ou l'un de ses sels a une masse moléculaire comprise entre 2 MDa et 4 MDa.

Dans un mode de réalisation, ledit polysaccharide ou l'un de ses sels a une masse moléculaire comprise entre 0,5 MDa et 2 MDa.

Dans un mode de réalisation, ledit acide polysaccharide ou l'un de ses sels a une masse moléculaire comprise entre 0,5 MDa et 1,5 MDa.

Dans un mode de réalisation, ledit acide hyaluronique ou l'un de ses sels a une masse moléculaire comprise entre 0,01 MDa et 10 MDa.

Dans un mode de réalisation, ledit acide hyaluronique ou l'un de ses sels a une masse moléculaire comprise entre 0,01 MDa et 5 MDa.

Dans un mode de réalisation, ledit acide hyaluronique ou l'un de ses sels a une masse moléculaire comprise entre 0,01 MDa et 3,5 MDa.

Dans un mode de réalisation, ledit acide hyaluronique ou l'un de ses sels a une masse moléculaire comprise entre 0,5 MDa et 3,5 MDa.

Dans un mode de réalisation, ledit acide hyaluronique ou l'un de ses sels a une masse moléculaire comprise entre 2,75 MDa et 3,25 MDa.

Dans un mode de réalisation, ledit acide hyaluronique ou l'un de ses sels a une masse moléculaire comprise entre 0,75 MDa et 1,25 MDa.

Dans un mode de réalisation, ledit acide hyaluronique ou l'un de ses sels a une masse moléculaire comprise entre 2 MDa et 5 MDa.

Dans un mode de réalisation, ledit acide hyaluronique ou l'un de ses sels a une masse moléculaire comprise entre 2 MDa et 4 MDa.

Dans un mode de réalisation, ledit acide hyaluronique ou l'un de ses sels a une masse moléculaire comprise entre 0,5 MDa et 2 MDa.

Dans un mode de réalisation, ledit acide hyaluronique ou l'un de ses sels a une masse moléculaire comprise entre 0,5 MDa et 1,5 MDa.

Dans un mode de réalisation, ledit héparosan ou l'un de ses sels a une masse moléculaire comprise entre 0,01 MDa et 10 MDa.

Dans un mode de réalisation, ledit héparosan ou l'un de ses sels a une masse moléculaire comprise entre 0,01 MDa et 5 MDa.

Dans un mode de réalisation, ledit héparosan ou l'un de ses sels a une masse moléculaire comprise entre 0,02 MDa et 3 MDa.

Dans un mode de réalisation, ledit héparosan ou l'un de ses sels a une masse moléculaire comprise entre 0,02 MDa et 2 MDa.

Pour la préparation des compositions selon l'invention, une ou plusieurs étapes de réticulation c) sont mis en œuvre entre un polysaccharide et au moins un agent réticulant.

Dans un mode de réalisation, le procédé de préparation des compositions selon l'invention est caractérisé en ce que la mise en présence dudit polysaccharide et de l'au moins un agent réticulant a lieu dans un solvant.

Dans un mode de réalisation, le procédé de préparation des compositions selon l'invention est caractérisé en ce que ledit au moins un agent réticulant de l'étape c) est choisi dans le groupe constitué d'un bis-époxy tel que l'éther d'éthylèneglycoldiglycidyle, le 1,4-butanediol diglycidyl éther (BDDE), le 1,2,3,4-diépoxybutane ou le 1,2,7,8-diépoxyoctane, d'une dialkylsulfone, de la divinylsulfone, du formaldéhyde, de l'épichlorohydrine ou bien encore du glutaraldéhyde, des carbodiimides tels que par exemple le 1-éthyl-3-[3-diméthylaminopropyl]carbodiimide hydrochloride (EDC), des trimétaphosphates, comme par exemple le trimétaphosphate de sodium, le trimétaphosphate de calcium, ou encore le trimétaphosphate de baryum.

Dans un mode de réalisation, le procédé de préparation des compositions selon l'invention est caractérisé en ce que ledit au moins un agent réticulant de l'étape c) est choisi dans le groupe constitué d'un bis-époxy tel que l'éther d'éthylèneglycoldiglycidyle, le 1,4-butanediol diglycidyl éther (BDDE), le 1,2,3,4-diépoxybutane ou le 1,2,7,8-diépoxyoctane, des trimétaphosphates, comme par exemple le trimétaphosphate de sodium, le trimétaphosphate de calcium, ou encore le trimétaphosphate de baryum.

Dans un mode de réalisation, le procédé de préparation des compositions selon l'invention est caractérisé en ce que ledit au moins un agent réticulant de l'étape c) est choisi dans le groupe constitué de l'éther d'éthylèneglycoldiglycidyle, du 1,4-butanediol diglycidyl éther (BDDE), du 1,2,3,4-diépoxybutane ou du 1,2,7,8-diépoxyoctane.

Dans un mode de réalisation, le procédé de préparation des compositions selon l'invention est caractérisé en ce que ledit au moins un agent réticulant de l'étape c) est choisi dans le groupe constitué des polyéthylèneglycol (PEG) difonctionnels portant un époxyde à chaque extrémité de la chaine de polymère.

Dans un mode de réalisation, le procédé de préparation des compositions selon l'invention est caractérisé en ce que ledit au moins un agent réticulant de l'étape c) est choisi dans le groupe constitué des polyéthylèneglycol (PEG) difonctionnels portant un groupe époxyde à chaque extrémité de la chaine de polymère choisi dans le groupe constitué par l'éther de polyéthylèneglycoldiglycidyle, l'éther de polypropylèneglycoldiglycidyle et l'éther de polytétraméthylèneglycoldiglycidyle.

Dans un mode de réalisation, le procédé de préparation des compositions selon l'invention est caractérisé en ce que ledit au moins un agent réticulant de l'étape c) est choisi dans le groupe constitué des trimétaphosphates, comme par exemple le trimétaphosphate de sodium, le trimétaphosphate de calcium, ou encore le trimétaphosphate de baryum.

Dans un mode de réalisation, le procédé de préparation des compositions selon l'invention est caractérisé en ce que ledit au moins un agent réticulant de l'étape c) est choisi dans le groupe constitué des époxydes, par exemple le 1,4-butanediol diglycidyl éther (BDDE), des épihalohydrines, de la divinylsulfone (DVS).

Dans un mode de réalisation, le procédé de préparation des compositions selon l'invention est caractérisé en ce que ledit au moins un agent réticulant de l'étape c) est la divinylsulfone (DVS).

Dans un mode de réalisation, le procédé de préparation des compositions selon l'invention est caractérisé en ce que ledit au moins un agent réticulant de l'étape c) est le 1,4-butanediol diglycidyl éther (BDDE).

Dans le cadre de la présente demande, le BDDE est particulièrement préféré.

Dans un mode de réalisation, le procédé de préparation des compositions selon l'invention est caractérisé en ce que ledit au moins un agent réticulant de l'étape c) est choisi parmi les composés portant deux fonctions choisies dans le groupe constitué des fonctions amines, alcoxyamines et hydrazides.

Dans un mode de réalisation, le procédé de préparation des compositions selon l'invention est caractérisé en ce que ledit au moins un agent réticulant de l'étape c) est choisi parmi les diamines.

Dans un mode de réalisation, la diamine utilisée comme agent réticulant de l'étape c) du procédé de préparation des compositions selon l'invention est choisie dans le groupe constitué du diaminotréhalose, du diaminosucrose, de la chitobiose, du diaminolactose et du diaminoraffinose.

Dans un mode de réalisation, la diamine utilisée comme agent réticulant de l'étape c) du procédé de préparation des compositions selon l'invention est le diaminotréhalose.

Dans un mode de réalisation, le procédé de préparation des compositions selon l'invention est caractérisé en ce que ledit au moins un agent réticulant de l'étape c) est choisi parmi les dialcoxyamines.

Dans un mode de réalisation, le procédé de préparation des compositions selon l'invention est caractérisé en ce que ledit au moins un agent réticulant de l'étape c) est choisi parmi les dihydrazides.

Dans un mode de réalisation, la dihydrazide utilisée comme agent réticulant de l'étape c) du procédé de préparation des compositions selon l'invention est l'acide adipique dihydrazide.

Dans un mode de réalisation, l'étape ou chacune des étapes de réticulation c) nécessite qu'on effectue préalablement une mise en solution dudit polysaccharide.

Dans un mode de réalisation, le polysaccharide est ajouté sous forme solide à une solution pour la mise en solution dudit polysaccharide.

Dans un mode de réalisation, une solution est ajoutée au polysaccharide sous forme solide pour la mise en solution dudit polysaccharide.

Dans un mode de réalisation, l'étape ou chacune des étapes de réticulation c) ne nécessite pas de mise en solution dudit polysaccharide.

La mise en solution du polysaccharide est effectuée par addition d'eau ou d'une solution aqueuse saline, par exemple une solution de tampon phosphate, par exemple PBS, ou par addition d'une solution de soude ou d'acide pour obtenir le pH compatible avec la mise en œuvre de l'étape ou de chacune des étapes de réticulation c).

Dans un mode de réalisation, le procédé d'obtention des compositions selon l'invention est caractérisé en ce qu'au plus tard lors de l'étape c), on effectue une étape d'ajustement du pH à un pH de réticulation.

L'ajustement du pH est effectué par ajout d'une solution acide minéral de préférence, par exemple acide chlorhydrique ou d'une base minérale de préférence, par exemple soude ou potasse, lesdits acides et bases étant ajoutés dans une quantité permettant d'atteindre le pH de réticulation visé.

Dans un mode de réalisation, lors de l'étape ou chacune des étapes de réticulation c), on effectue une étape d'ajustement du pH à un pH de réticulation adapté audit agent réticulant.

Dans un mode de réalisation, au plus tard lors de l'étape de réticulation ou de chacune des étapes de réticulation c), on effectue une étape d'ajustement du pH à un pH de réticulation supérieur à 10.

Dans un mode de réalisation, au plus tard lors de l'étape de réticulation ou de chacune des étapes de réticulation c), on effectue une étape d'ajustement du pH à un pH de réticulation inférieur à 3.

Dans un mode de réalisation, le pH de réticulation visé lors de l'étape de réticulation ou de chacune des étapes de réticulation c) est obtenu par ajout d'une solution de soude de concentration au plus 0,25 N ou 1% en masse.

Dans un mode de réalisation, au plus tard lors de l'étape de réticulation ou de chacune des étapes de réticulation c), on effectue une étape d'ajustement du pH à un pH de réticulation supérieur à 10, ledit agent réticulant étant le BDDE.

Dans un mode de réalisation, au plus tard lors de l'étape de réticulation ou de chacune des étapes de réticulation c), on effectue une étape d'ajustement du pH à un pH de réticulation inférieur à 3, ledit agent réticulant étant le BDDE.

Dans un mode de réalisation, au plus tard lors de l'étape de réticulation ou de chacune des étapes de réticulation c), on effectue une étape d'ajustement du pH à un pH de réticulation, ledit pH de réticulation étant supérieur à 10.

La réticulation débute lorsque les 3 conditions suivantes sont réunies : présence du polysaccharide, présence de l'agent réticulant, milieu réactionnel à un pH approprié.

Dans un mode de réalisation, l'étape de réticulation ou chacune des étapes de réticulation c) est caractérisée en ce que l'initiation de la réticulation est provoquée par l'ajout dudit agent réticulant.

Dans un mode de réalisation, l'étape de réticulation ou chacune des étapes de réticulation c) est caractérisée en ce que l'initiation de la réticulation est provoquée par l'ajout dudit polysaccharide.

Dans un mode de réalisation, l'étape de réticulation ou chacune des étapes de réticulation c) est caractérisée en ce que l'initiation de la réticulation est provoquée par l'application d'un pH de réticulation.

Dans un mode de réalisation, le procédé de préparation des compositions selon l'invention est caractérisé en ce qu'après l'étape c), on effectue une étape d'ajustement du pH à un pH compris entre 6 et 8.

En fonction du pH du milieu réactionnel à l'issue de la réaction de réticulation de l'étape c), l'ajustement du pH est effectué par ajout d'une solution d'acide minéral de préférence, par exemple acide chlorhydrique ou d'une base minérale de préférence, par exemple soude ou potasse, lesdits acides et bases étant ajoutés dans une quantité permettant d'atteindre un pH compris entre 6 et 8.

Dans un mode de réalisation, le procédé de préparation des compositions selon l'invention est caractérisé en ce qu'après l'étape c), on effectue une étape d'ajustement du pH à un pH compris entre 6 et 8 par ajout d'au moins un acide étant l'acide chlorhydrique (HCl).

Le procédé de préparation des compositions selon l'invention est caractérisé en ce que l'étape ou chacune des étapes de réticulation c) est mise en œuvre à température constante ou à température variable de façon linéaire ou par paliers, ladite température constante ou variable étant inférieure ou égale à 50 °C.

Le procédé de préparation des compositions selon l'invention est caractérisé en ce que l'étape ou chacune des étapes de réticulation c) est mise en œuvre à température constante ou à température variable de façon linéaire ou par paliers, ladite température constante ou variable étant inférieure ou égale à 30 °C.

Le procédé de préparation des compositions selon l'invention est caractérisé en ce que l'étape ou chacune des étapes de réticulation c) est mise en œuvre à température constante ou à température variable de façon linéaire ou par paliers, ladite température constante ou variable étant inférieure ou égale à 20 °C.

Le procédé de préparation des compositions selon l'invention est caractérisé en ce que l'étape ou chacune des étapes de réticulation c) est mise en œuvre à température constante ou à température variable de façon linéaire ou par paliers, ladite température constante ou variable étant inférieure ou égale à 15 °C.

Le procédé de préparation des compositions selon l'invention est caractérisé en ce que l'étape ou chacune des étapes de réticulation c) est mise en œuvre à température constante ou à température variable de façon linéaire ou par paliers, ladite température constante ou variable étant inférieure ou égale à 10 °C.

Le procédé de préparation des compositions selon l'invention est caractérisé en ce que l'étape ou chacune des étapes de réticulation c) est mise en œuvre à température constante ou à température variable de façon linéaire ou par paliers, ladite température constante ou variable étant inférieure ou égale à 5 °C.

On entend par température de solidification du milieu réactionnel, la température à laquelle le milieu devient solide. Pour un milieu aqueux, cette température se situera à une température de 0 °C, ou légèrement inférieure en fonction de la concentration en sels dudit milieu.

Dans un mode de réalisation, l'étape ou chacune des étapes de réticulation c) est mise en œuvre à température constante ou à température variable de façon linéaire ou par paliers, ladite température constante ou variable étant comprise entre la température de solidification et 10 °C.

Dans un mode de réalisation, l'étape ou chacune des étapes de réticulation c) est mise en œuvre à température constante ou à température variable de façon linéaire ou par paliers, ladite température constante ou variable étant comprise entre la température de solidification et 5 °C.

Dans un mode de réalisation, l'étape ou chacune des étapes de réticulation c) est mise en œuvre à température constante à 50 °C.

Dans un mode de réalisation, l'étape ou chacune des étapes de réticulation c) est mise en œuvre à température constante à 30 °C.

Dans un mode de réalisation, l'étape ou chacune des étapes de réticulation c) est mise en œuvre à température constante à 20 °C.

Dans un mode de réalisation, l'étape ou chacune des étapes de réticulation c) est mise en œuvre à température constante à 15 °C.

Dans un mode de réalisation, l'étape ou chacune des étapes de réticulation c) est mise en œuvre à température constante à 9 °C.

Dans un mode de réalisation, l'étape ou chacune des étapes de réticulation c) est mise en œuvre à température constante à 5 °C.

Dans un mode de réalisation, l'étape ou chacune des étapes de réticulation c) est mise en œuvre à température constante à 2 °C.

Dans un mode de réalisation, avant l'étape de réticulation ou chacune des étapes de réticulation c), on effectue une étape de refroidissement à la température de réticulation.

Dans un mode de réalisation, le procédé de préparation des compositions selon l'invention est caractérisé en ce que ledit au moins un agent réticulant de l'étape c) est le 1,4-butanediol diglycidyl éther (BDDE), et ladite étape c) est réalisée à un pH supérieur à 10 et à une température inférieure ou égale à 50 °C.

Dans un mode de réalisation, le procédé de préparation des compositions selon l'invention est caractérisé en ce que ledit au moins un agent réticulant de l'étape c) est le 1,4-butanediol diglycidyl éther (BDDE), et ladite étape c) est réalisée à un pH supérieur à 10 et à une température inférieure ou égale à 30 °C.

Dans un mode de réalisation, le procédé de préparation des compositions selon l'invention est caractérisé en ce que ledit au moins un agent réticulant de l'étape c) est le 1,4-butanediol diglycidyl éther (BDDE), et ladite étape c) est réalisée à un pH supérieur à 10 et à une température inférieure ou égale à 20 °C.

Dans un mode de réalisation, le procédé de préparation des compositions selon l'invention est caractérisé en ce que ledit au moins un agent réticulant de l'étape c) est le 1,4-butanediol diglycidyl éther (BDDE), et ladite étape c) est réalisée à un pH supérieur à 10 et à une température inférieure ou égale à 15 °C.

Dans un mode de réalisation, le procédé de préparation des compositions selon l'invention est caractérisé en ce que ledit au moins un agent réticulant de l'étape c) est le 1,4-butanediol diglycidyl éther (BDDE), et ladite étape c) est réalisée à un pH supérieur à 10 et à une température inférieure ou égale à 10 °C.

Dans un mode de réalisation, le procédé de préparation des compositions selon l'invention est caractérisé en ce que ledit au moins un agent réticulant de l'étape c) est le 1,4-butanediol diglycidyl éther (BDDE), et ladite étape c) est réalisée à un pH supérieur à 10 et à une température inférieure ou égale à 5 °C.

Dans un mode de réalisation, le procédé de préparation des compositions selon l'invention est caractérisé en ce que ledit au moins un agent réticulant de l'étape c) est le 1,4-butanediol diglycidyl éther (BDDE), et ladite étape c) est réalisée à un pH supérieur à 10 et à une température inférieure ou égale à 2 °C.

Dans un mode de réalisation, le procédé de préparation des compositions selon l'invention est caractérisé en ce que l'étape c) de réticulation a une durée comprise entre 10 min et 26 heures.

Dans un mode de réalisation, le procédé de préparation des compositions selon l'invention est caractérisé en ce que l'étape c) de réticulation a une durée comprise entre 10 min et 18 heures.

Dans un mode de réalisation, le procédé de préparation des compositions selon l'invention est caractérisé en ce que l'étape c) de réticulation a une durée comprise entre 10 min et 12 heures.

Dans un mode de réalisation, le procédé de préparation des compositions selon l'invention est caractérisé en ce que l'étape c) de réticulation a une durée comprise entre 10 min et 5 heures.

Dans un mode de réalisation, le procédé de préparation des compositions selon l'invention est caractérisé en ce que l'étape c) de réticulation a une durée comprise entre 10 min et 3 heures.

Dans un mode de réalisation, le procédé de préparation des compositions selon l'invention est caractérisé en ce que l'étape c) de réticulation a une durée comprise entre 30 min et 3 heures.

Dans un mode de réalisation, le procédé de préparation des compositions selon l'invention est caractérisé en ce que l'étape de réticulation c) est mise en œuvre à 2 °C pendant une durée de 24 heures.

Dans un mode de réalisation, le procédé de préparation des compositions selon l'invention est caractérisé en ce que ledit au moins un agent réticulant de l'étape c) est le 1,4-butanediol diglycidyl éther (BDDE), et ladite étape c) est réalisée à un pH supérieur à 10, à une température de 2 °C pendant une durée de 24 heures.

Dans un mode de réalisation, le procédé de préparation des compositions selon l'invention est caractérisé en ce que l'étape de réticulation c) est mise en œuvre à 9 °C pendant une durée de 3 heures.

Dans un mode de réalisation, le procédé de préparation des compositions selon l'invention est caractérisé en ce que ledit au moins un agent réticulant de l'étape c) est le 1,4-butanediol diglycidyl éther (BDDE), et ladite étape c) est réalisée à un pH supérieur à 10, à une température de 9 °C pendant une durée de 3 heures.

Lorsque plusieurs réticulations successives sont réalisées à l'étape c) du procédé de préparation des compositions selon l'invention, les durées mentionnées sont les durées totales (somme des durées des réticulations successives).

Dans un mode de réalisation, lors de l'étape c), la mise en œuvre de la ou des étape(s) de réticulation en présence dudit polysaccharide et dudit agent réticulant a lieu dans un milieu dans lequel ledit polysaccharide est hydraté et/ou gonflé par addition d'eau ou d'une solution aqueuse saline, par exemple une solution de tampon phosphate, par exemple PBS.
Lors de l'étape c) du procédé de préparation des compositions selon l'invention, le taux de réticulation (X) peut être calculé de manière théorique au moyen de la formule suivante :

$$X = \frac{\text{nombre de moles d'agent réticulant introduites dans le milieu réactionnel}}{\text{nombre de moles d'unités de répétition (motif disaccharidique) introduites dans le milieu réactionnel}}$$

Ainsi, par exemple si un milieu comprend 100 motifs disaccharidiques, et que ledit milieu comprend également 10 molécules d'agent réticulant, alors le taux de réticulation (X) sera le suivant : X = 10/100 = 0,1. Ce taux de réticulation n'est donc influencé ni par le degré de polymérisation, ni par la masse moléculaire du polysaccharide choisi, ni par la proportion d'agent réticulant qui réagit effectivement avec au moins une fonction du polysaccharide. Il s'agit d'une détermination théorique prenant en compte uniquement les quantités d'agent réticulant et d'unités de répétition mises en présence.
Dans un mode de réalisation, le taux de réticulation X est compris entre 0,001 et 0,20.
Dans un mode de réalisation, le taux de réticulation X est compris entre 0,01 et 0,15.
Dans un mode de réalisation, le taux de réticulation X est compris entre 0,01 et 0,12.
Dans un mode de réalisation, le taux de réticulation X est compris entre 0,03 et 0,10.
Dans un mode de réalisation, le taux de réticulation X est compris entre 0,04 et 0,08.
La réticulation peut également être appréciée, a posteriori (après la réticulation), au moyen du degré de modification (Mod). Le Mod tient donc compte, à l'inverse du taux de réticulation X, de la proportion d'agent réticulant qui réagit effectivement avec au moins une fonction du polysaccharide.
Le degré de modification peut être exprimé comme suit :

$$\text{Mod (\%)} = \frac{\text{nombre de moles d'agent réticulant liées par au moins une liaison covalente à au moins un motif disaccharidique}}{\text{nombre de moles d'unités de répétition présentes dans le milieu réactionnel}} * 100$$

L'unité de répétition (ou monomère) est, lorsque le polysaccharide est l'acide hyaluronique, un motif disaccharidique.
La détermination des valeurs au numérateur et au dénominateur dépend du polysaccharide choisi et de l'agent réticulant choisi, et sont bien connues de l'homme du métier. Par exemple, dans le cas particulier d'une formulation à base d'acide hyaluronique réticulé par du BDDE, la méthode décrite dans la publication L. Nord, A. Emilson, C. Sturesson, A.H. Kenne, Degree of Modification of Hyaluronic Acid Dermal Fillers, 18th Congress of the EADV, Berlin, 2009 peut être utilisée.
Dans le cas particulier d'une formulation à base d'acide hyaluronique réticulé par du BDDE, le degré de modification peut être exprimé comme suit :

$$\text{Mod (\%)} = \frac{\text{nombre de moles de BDDE liées par au moins une liaison covalente à au moins un motif disaccharidique d'acide hyaluronique}}{\text{nombre de moles d'unités de répétition (motif disaccharidique d'acide hyaluronique) présentes dans le milieu réactionnel}} * 100$$

Par exemple, une formulation à base d'acide hyaluronique réticulée par du BDDE ayant un Mod de 1% signifie qu'elle présente une molécule de BDDE (monoliée ou doublement liée) pour 100 motifs disaccharidiques.
Dans un mode de réalisation, le degré de modification dudit polysaccharide réticulé est inférieur à 5%.
Dans un mode de réalisation, le degré de modification dudit polysaccharide réticulé est inférieur à 4%.
Dans un mode de réalisation, le degré de modification dudit polysaccharide réticulé est inférieur à 3%.
Dans un mode de réalisation, le degré de modification dudit polysaccharide réticulé est inférieur à 2%.
Dans un mode de réalisation, lors de l'étape c), mise en œuvre de la ou des étape(s) de réticulation en présence dudit polysaccharide et dudit agent réticulant la concentration en polysaccharide est d'au moins 1% en masse, par rapport à la masse totale du milieu réactionnel de réticulation.
Dans un mode de réalisation, lors de l'étape c), mise en œuvre de la ou des étape(s) de réticulation en présence

dudit polysaccharide et dudit agent réticulant la concentration en polysaccharide est d'au moins 5% en masse, par rapport à la masse totale du milieu réactionnel de réticulation.

Dans un mode de réalisation, lors de l'étape c), mise en œuvre de la ou des étape(s) de réticulation en présence dudit polysaccharide et dudit agent réticulant la concentration en polysaccharide est d'au moins 10% en masse, par rapport à la masse totale du milieu réactionnel de réticulation.

Dans un mode de réalisation, lors de l'étape c), mise en œuvre de la ou des étape(s) de réticulation en présence dudit polysaccharide et dudit agent réticulant la concentration en polysaccharide est d'au moins 15% en masse, par rapport à la masse totale du milieu réactionnel de réticulation.

Dans un mode de réalisation, lors de l'étape c), mise en œuvre de la ou des étape(s) de réticulation en présence dudit polysaccharide et dudit agent réticulant la concentration en polysaccharide est comprise entre 1 et 60% en masse, par rapport à la masse totale du milieu réactionnel de réticulation.

Dans un mode de réalisation, lors de l'étape c), mise en œuvre de la ou des étape(s) de réticulation en présence dudit polysaccharide et dudit agent réticulant la concentration en polysaccharide est comprise entre 5 et 60% en masse, par rapport à la masse totale du milieu réactionnel de réticulation.

Dans un mode de réalisation, lors de l'étape c), mise en œuvre de la ou des étape(s) de réticulation en présence dudit polysaccharide et dudit agent réticulant la concentration en polysaccharide est comprise entre 10 et 60% en masse, par rapport à la masse totale du milieu réactionnel de réticulation.

Dans un mode de réalisation, lors de l'étape c), mise en œuvre de la ou des étape(s) de réticulation en présence dudit polysaccharide et dudit agent réticulant, la concentration en polysaccharide est comprise entre 10 et 40% en masse, par rapport à la masse totale du milieu réactionnel.

Dans un mode de réalisation, lors de l'étape c), mise en œuvre de la ou des étape(s) de réticulation en présence dudit polysaccharide et dudit agent réticulant la concentration en polysaccharide est comprise entre 10 et 25% en masse, par rapport à la masse totale du milieu réactionnel.

Dans un mode de réalisation, lors de l'étape c), mise en œuvre de la ou des étape(s) de réticulation en présence dudit polysaccharide et dudit agent réticulant la concentration en polysaccharide est comprise entre 15 et 25% en masse, par rapport à la masse totale du milieu réactionnel.

Dans un mode de réalisation, lors de l'étape c), mise en œuvre de la ou des étape(s) de réticulation en présence d'acide hyaluronique, ou l'un de ses sels biologiquement acceptables, seuls ou en mélange, et dudit agent réticulant dans un milieu réactionnel de réticulation, la concentration en acide hyaluronique est comprise entre 1 et 60% en masse, par rapport à la masse totale du milieu réactionnel.

Dans un mode de réalisation, lors de l'étape c), mise en œuvre de la ou des étape(s) de réticulation en présence d'acide hyaluronique, ou l'un de ses sels biologiquement acceptables, seuls ou en mélange, et dudit agent réticulant dans un milieu réactionnel de réticulation, la concentration en acide hyaluronique est comprise entre 5 et 60% en masse, par rapport à la masse totale du milieu réactionnel.

Dans un mode de réalisation, lors de l'étape c), mise en œuvre de la ou des étape(s) de réticulation en présence d'acide hyaluronique, ou l'un de ses sels biologiquement acceptables, seuls ou en mélange, et dudit agent réticulant dans un milieu réactionnel de réticulation, la concentration en acide hyaluronique est comprise entre 10 et 60% en masse, par rapport à la masse totale du milieu réactionnel.

Dans un mode de réalisation, lors de l'étape c), mise en œuvre de la ou des étape(s) de réticulation en présence d'acide hyaluronique, ou l'un de ses sels biologiquement acceptables, seuls ou en mélange, et dudit agent réticulant la concentration en acide hyaluronique est comprise entre 10 et 40% en masse, par rapport à la masse totale du milieu réactionnel.

Dans un mode de réalisation, lors de l'étape c), mise en œuvre de la ou des étape(s) de réticulation en présence d'acide hyaluronique, ou l'un de ses sels biologiquement acceptables, seuls ou en mélange, et dudit agent réticulant la concentration en acide hyaluronique est comprise entre 10 et 25% en masse, par rapport à la masse totale du milieu réactionnel.

Dans un mode de réalisation, lors de l'étape c), mise en œuvre de la ou des étape(s) de réticulation en présence d'acide hyaluronique, ou l'un de ses sels biologiquement acceptables, seuls ou en mélange, et dudit agent réticulant la concentration en acide hyaluronique est comprise entre 15 et 25% en masse, par rapport à la masse totale du milieu réactionnel.

Dans un mode de réalisation, lors de l'étape c), mise en œuvre de la ou des étape(s) de réticulation en présence d'héparosan, ou l'un de ses sels biologiquement acceptables, seuls ou en mélange, et dudit agent réticulant dans un milieu réactionnel de réticulation, la concentration en héparosan est comprise entre 1 et 60% en masse, par rapport à la masse totale du milieu réactionnel.

Dans un mode de réalisation, lors de l'étape c), mise en œuvre de la ou des étape(s) de réticulation en présence d'héparosan, ou l'un de ses sels biologiquement acceptables, seuls ou en mélange, et dudit agent réticulant dans un milieu réactionnel de réticulation, la concentration en héparosan est comprise entre 5 et 60% en masse, par rapport à la masse totale du milieu réactionnel.

Dans un mode de réalisation, lors de l'étape c), mise en œuvre de la ou des étape(s) de réticulation en présence d'héparosan, ou l'un de ses sels biologiquement acceptables, seuls ou en mélange, et dudit agent réticulant dans un milieu réactionnel de réticulation, la concentration en héparosan est comprise entre 10 et 60% en masse, par rapport à la masse totale du milieu réactionnel.

Dans un mode de réalisation, lors de l'étape c), mise en œuvre de la ou des étape(s) de réticulation en présence d'héparosan, ou l'un de ses sels biologiquement acceptables, seuls ou en mélange, et dudit agent réticulant la concentration en héparosan est comprise entre 10 et 40% en masse, par rapport à la masse totale du milieu réactionnel.

Dans un mode de réalisation, lors de l'étape c), mise en œuvre de la ou des étape(s) de réticulation en présence d'héparosan, ou l'un de ses sels biologiquement acceptables, seuls ou en mélange, et dudit agent réticulant la concentration en héparosan est comprise entre 10 et 25% en masse, par rapport à la masse totale du milieu réactionnel.

Dans un mode de réalisation, lors de l'étape c), mise en œuvre de la ou des étape(s) de réticulation en présence d'héparosan, ou l'un de ses sels biologiquement acceptables, seuls ou en mélange, et dudit agent réticulant la concentration en héparosan est comprise entre 15 et 25% en masse, par rapport à la masse totale du milieu réactionnel.

Dans un mode de réalisation, lors de l'étape c), mise en œuvre de la ou des étape(s) de réticulation en présence dudit polysaccharide et dudit agent réticulant le milieu réactionnel de réticulation comprend de la soude (NaOH).

Dans un mode de réalisation, lors de l'étape c), mise en œuvre de la ou des étape(s) de réticulation en présence dudit polysaccharide et dudit agent réticulant, la concentration en soude est comprise entre 0,5 et 1,5% en masse, par rapport à la masse totale du milieu réactionnel.

Dans un mode de réalisation, lors de l'étape c), mise en œuvre de la ou des étape(s) de réticulation en présence dudit polysaccharide et dudit agent réticulant, la concentration en soude est comprise entre 0,5 et 1% en masse, par rapport à la masse totale du milieu réactionnel.

Dans un mode de réalisation, lors de l'étape c), mise en œuvre de la ou des étape(s) de réticulation en présence dudit polysaccharide et dudit agent réticulant la concentration en soude est comprise entre 0,7 et 0,9% en masse, par rapport à la masse totale du milieu réactionnel.

Dans un mode de réalisation, lors de l'étape c), mise en œuvre de la ou des étape(s) de réticulation en présence dudit polysaccharide et dudit agent réticulant le milieu réactionnel de réticulation comprend de l'acide chlorhydrique (HCl).

Dans un mode de réalisation, lors de l'étape c), mise en œuvre de la ou des étape(s) de réticulation en présence dudit polysaccharide et dudit agent réticulant, la concentration en acide chlorhydrique est comprise entre 0,001 et 0,05 M dans le milieu réactionnel.

Dans un mode de réalisation, l'étape c) de réticulation comporte au moins une étape d'activation de fonctions chimiques portées par le polysaccharide.

Dans un mode de réalisation, l'étape c) de réticulation comporte au moins une étape d'activation de fonctions acides carboxyliques portées par le polysaccharide.

Dans un mode de réalisation, l'étape d'activation des fonctions acides carboxyliques portées par le polysaccharide est effectuée à partir d'un agent de couplage utilisé en chimie des peptides.

Dans un mode de réalisation, l'étape d'activation des fonctions acides carboxyliques portées par le polysaccharide est effectuée à partir d'un agent de couplage utilisé en chimie des peptides choisi dans le groupe des agents de couplage de type triazine, carbodiimide, imidazolium ainsi que Oxyma et COMU.

Dans un mode de réalisation, l'agent de couplage de type triazine est choisi dans le groupe constitué par le chlorure de 4-(4,6-diméthoxy[1.3.5]triazin-2-yl)-4-méthylmorpholinium (DMTMM) et la 2-Chloro-4,6-diméthoxy-1,3,5-triazine (CMT).

Dans un mode de réalisation, l'agent de couplage est le chlorure de 4-(4,6-diméthoxy[1.3.5]triazin-2-yl)-4-méthylmorpholinium (DMTMM).

Dans un mode de réalisation, le procédé de préparation des compositions selon l'invention est caractérisé en ce qu'avant l'étape d), on effectue une étape d'élimination dudit agent réticulant.

Dans un mode de réalisation, le procédé de préparation des compositions selon l'invention est caractérisé en ce qu'avant l'étape d), on effectue une étape de purification du polysaccharide réticulé.

Dans un mode de réalisation, le procédé de préparation des compositions selon l'invention est caractérisé en ce qu'avant l'étape d), on effectue une étape de purification du polysaccharide réticulé par dialyse.

Dans un mode de réalisation, le procédé de préparation des compositions selon l'invention est caractérisé en ce qu'avant l'étape d), on effectue une étape de purification du polysaccharide réticulé par dialyse au moyen d'une solution ou d'un solvant de dialyse choisi dans le groupe constitué des tampons phosphates par exemple PBS et de l'eau.

Dans un mode de réalisation, le procédé de préparation des compositions selon l'invention est caractérisé en ce qu'avant l'étape d), on effectue une étape de purification du polysaccharide réticulé par lavages.

Dans un mode de réalisation, on effectue une étape de purification du polysaccharide réticulé par lavages au moyen d'une solution ou d'un solvant choisi dans le groupe constitué des tampons phosphates par exemple PBS et de l'eau.

Dans un mode de réalisation, le procédé de préparation des compositions selon l'invention est caractérisé en

ce qu'avant l'étape d), on effectue une étape de purification du polysaccharide réticulé par précipitation sous forme salifiée.

Dans un mode de réalisation, le procédé de préparation des compositions selon l'invention est caractérisé en ce qu'avant l'étape d), on effectue une étape de purification du polysaccharide réticulé par précipitation à partir d'une solution obtenue après ajout de sel PBS ou NaCl.

Dans un mode de réalisation, l'étape de purification du polysaccharide réticulé par précipitation est effectuée par ajout d'au moins un solvant organique au polysaccharide réticulé.

Dans un mode de réalisation, l'au moins un solvant organique ajouté au polysaccharide réticulé dans l'étape de purification par précipitation est choisi dans le groupe des alcools.

Dans un mode de réalisation, l'alcool ajouté au polysaccharide réticulé dans l'étape de purification par précipitation est l'éthanol.

Dans un mode de réalisation, l'étape de purification du polysaccharide réticulé par précipitation a lieu dans un mélange hydroalcoolique.

Dans un mode de réalisation, le polysaccharide réticulé précipité est isolé par filtration.

Dans un mode de réalisation, le polysaccharide réticulé précipité et isolé par filtration est lavé.

Dans un mode de réalisation, le polysaccharide réticulé précipité et isolé par filtration est séché.

Dans un mode de réalisation, le polysaccharide précipité réticulé et isolé par filtration est séché sous atmosphère et température ambiante.

Dans un mode de réalisation, le polysaccharide réticulé précipité et récupéré par filtration est séché sous vide à température ambiante.

Dans un mode de réalisation, le polysaccharide réticulé est lyophilisé.

Le procédé de préparation des compositions selon l'invention comprend au moins une étape de rupture de liaisons glycosidiques, étape e), mise en œuvre sur au moins un polysaccharide réticulé et permettant d'obtenir le composé macromoléculaire selon l'invention.

Dans un mode de réalisation, l'étape e) de rupture de liaisons glycosidiques mise en œuvre sur au moins un polysaccharide réticulé repose sur un mécanisme de dégradation contrôlée.

Dans un mode de réalisation, l'étape e) de rupture de liaisons glycosidiques mise en œuvre sur au moins un polysaccharide réticulé est effectuée au moyen d'un traitement choisi dans le groupe des traitements chimiques, des traitements par des enzymes, des traitements par radiations et des traitements thermiques.

Dans un mode de réalisation, l'étape e) de rupture de liaisons glycosidiques mise en œuvre sur au moins un polysaccharide réticulé est effectuée au moyen d'un traitement chimique.

Dans un mode de réalisation, ce traitement chimique consiste à exposer l'au moins un polysaccharide réticulé à une solution aqueuse à pH basique.

Dans un mode de réalisation, la solution aqueuse à pH basique est une solution aqueuse d'hydroxyde de sodium (soude).

Dans un mode de réalisation, ce traitement chimique consiste à exposer l'au moins un polysaccharide réticulé à une solution aqueuse à pH acide.

Dans un mode de réalisation, la solution aqueuse à pH acide contient un acide choisi dans le groupe constitué de l'acide acétique, de l'acide chlorhydrique, de l'acide sulfurique et de l'acide phosphorique.

Dans un mode de réalisation, la solution aqueuse à pH acide est une solution aqueuse d'acide acétique.

Dans un mode de réalisation, la solution aqueuse à pH acide est une solution aqueuse d'acide chlorhydrique.

Dans un mode de réalisation, la solution aqueuse à pH acide est une solution aqueuse d'acide sulfurique.

Dans un mode de réalisation, la solution aqueuse à pH acide est une solution aqueuse d'acide phosphorique.

Dans un mode de réalisation, ce traitement chimique consiste à exposer l'au moins un polysaccharide réticulé à un agent oxydant.

Dans un mode de réalisation, l'agent oxydant est choisi dans le groupe constitué de l'eau oxygénée et de l'hypochlorite de sodium.

Dans un mode de réalisation, l'agent oxydant est l'eau oxygénée.

Dans un mode de réalisation, l'agent oxydant est l'hypochlorite de sodium.

Dans un mode de réalisation, ce traitement chimique consiste à faire subir au polysaccharide réticulé un procédé de dépolymérisation réductrice oxydative.

Dans un mode de réalisation, le procédé de dépolymérisation réductrice oxydative est effectué en présence d'au moins un des composés Fe2+, Fe3+, acide ascorbique et $H_2O_2$

Dans un mode de réalisation, ce traitement chimique de l'au moins un polysaccharide réticulé s'effectue sans chauffage, à température ambiante.

Dans un mode de réalisation, ce traitement chimique de l'au moins un polysaccharide réticulé s'effectue avec chauffage.

Dans un mode de réalisation, ce traitement chimique de l'au moins un polysaccharide réticulé s'effectue à basse température, par utilisation d'un système de refroidissement.

Dans un mode de réalisation, l'étape e) de rupture de liaisons glycosidiques mise en œuvre sur au moins un polysaccharide réticulé est effectuée au moyen d'un traitement par au moins une enzyme.

Dans un mode de réalisation, l'étape e) de rupture de liaisons glycosidiques mise en œuvre sur au moins un polysaccharide réticulé est effectuée au moyen d'un traitement par un mélange d'enzymes.

Dans un mode de réalisation, l'étape e) de rupture de liaisons glycosidiques mise en œuvre sur au moins un polysaccharide réticulé est effectuée au moyen d'un traitement par au moins une enzyme de type hydrolase.

Dans un mode de réalisation, l'étape e) de rupture de liaisons glycosidiques mise en œuvre sur au moins un polysaccharide réticulé est effectuée au moyen d'un traitement par au moins une enzyme de type lyase.

Dans un mode de réalisation, l'enzyme utilisée dans l'étape e) de rupture de liaisons glycosidiques mise en œuvre sur au moins un polysaccharide réticulé est choisi dans le groupe des hyaluronidases.

Dans un mode de réalisation, l'enzyme hyaluronidase utilisée dans l'étape e) de rupture de liaisons glycosidiques mise en œuvre sur au moins un polysaccharide réticulé est choisi dans le groupe constitué des enzymes HYAL1 et HYAL2.

Dans un mode de réalisation, l'enzyme hyaluronidase utilisée dans l'étape e) de rupture de liaisons glycosidiques mise en œuvre sur au moins un polysaccharide réticulé est HYAL1.

Dans un mode de réalisation, l'enzyme hyaluronidase utilisée dans l'étape e) de rupture de liaisons glycosidiques mise en œuvre sur au moins un polysaccharide réticulé est HYAL2.

Dans un mode de réalisation, l'enzyme utilisée dans l'étape e) de rupture de liaisons glycosidiques mise en œuvre sur au moins un polysaccharide réticulé est une enzyme choisie dans le groupe des enzymes bactériennes.

Dans un mode de réalisation, l'enzyme utilisée dans l'étape e) de rupture de liaisons glycosidiques mise en œuvre sur au moins un polysaccharide réticulé est une enzyme choisie dans le groupe des enzymes bactériennes constitué par les N-acétylhexosaminidases.

Dans un mode de réalisation, l'enzyme N-acétylhexosaminidase est choisi dans le groupe constitué de chondroïtinase ABC (CASE) et chondroïtinase AC.

Dans un mode de réalisation, l'enzyme N-acétylhexosaminidase est chondroïtinase ABC.

Dans un mode de réalisation, l'enzyme N-acétylhexosaminidase est chondroïtinase AC.

Dans un mode de réalisation, l'étape e) de rupture de liaisons glycosidiques mise en œuvre sur au moins un polysaccharide réticulé est effectuée au moyen d'un traitement par radiations.

Dans un mode réalisation, ce traitement par radiations consiste en une exposition de l'au moins un polysaccharide réticulé à un rayonnement gamma.

Dans un mode réalisation, ce traitement par radiations consiste en une exposition de l'au moins un polysaccharide réticulé à un rayonnement béta.

Dans un mode réalisation, ce traitement par radiations consiste en une exposition de l'au moins un polysaccharide réticulé à un faisceau d'électrons accélérés (électrons beam).

Dans un mode de réalisation, l'étape e) de rupture de liaisons glycosidiques mise en œuvre sur au moins un polysaccharide réticulé est effectuée au moyen d'un traitement thermique.

Dans un mode de réalisation, ce traitement thermique est une étape d'exposition de l'au moins un polysaccharide réticulé à une température d'au moins 60 °C pendant au moins 2 heures.

Dans un mode de réalisation, ce traitement thermique est une étape d'exposition de l'au moins un polysaccharide réticulé à la chaleur humide.

Dans un mode de réalisation, ce traitement thermique consistant à exposer l'au moins un polysaccharide réticulé à la chaleur humide est effectué par autoclavage à la vapeur.

Dans un mode de réalisation, l'autoclavage à la vapeur est effectué entre 120 °C et 130 °C.

Dans un mode de réalisation, l'autoclavage à la vapeur est effectué avec un équivalent temps final (F0) d'au moins 30 minutes.

Dans un mode de réalisation, l'autoclavage à la vapeur est effectué avec un équivalent temps final (F0) d'au moins 50 minutes.

Dans un mode de réalisation, l'autoclavage à la vapeur est effectué avec un équivalent temps final (F0) d'au moins 90 minutes.

Dans un mode de réalisation, ce traitement thermique consiste à exposer l'au moins un polysaccharide réticulé à la chaleur sèche.

Dans un mode de réalisation, l'étape e) de rupture de liaisons glycosidiques mise en œuvre sur au moins un polysaccharide réticulé est effectuée au moyen d'un traitement à haute pression.

Dans un mode de réalisation, l'étape e) de rupture de liaisons glycosidiques est mise en œuvre sur au moins un polysaccharide réticulé purifié.

Dans un mode de réalisation, l'étape e) de rupture de liaisons glycosidiques est mise en œuvre sur au moins un polysaccharide réticulé non purifié.

Dans un mode de réalisation, le procédé de préparation des compositions selon l'invention est caractérisé en ce qu'après l'étape e), on effectue une étape de purification du composé macromoléculaire obtenu.

Dans un mode de réalisation, l'étape de purification du composé macromoléculaire est une étape d'élimination dudit agent réticulant.

Dans un mode de réalisation, le procédé de préparation des compositions selon l'invention est caractérisé en ce qu'après l'étape e), on effectue une étape de purification par dialyse.

Dans un mode de réalisation, le procédé de préparation des compositions selon l'invention est caractérisé en ce qu'après l'étape e), on effectue une étape de purification de la solution de composé macromoléculaire par dialyse au moyen d'une solution ou d'un solvant de dialyse choisi dans le groupe constitué des tampons phosphates par exemple PBS et de l'eau.

Dans un mode de réalisation, le procédé de préparation des compositions selon l'invention est caractérisé en ce qu'après l'étape e), on effectue une étape de purification de la solution de composé macromoléculaire par lavages.

Dans un mode de réalisation, on effectue une étape de purification de la solution de composé macromoléculaire par lavages au moyen d'une solution ou d'un solvant choisi dans le groupe constitué des tampons phosphates par exemple PBS et de l'eau.

Dans un mode de réalisation, le procédé de préparation des compositions selon l'invention est caractérisé en ce qu'après l'étape e), on effectue une étape de purification par filtration tangentielle.

Dans un mode de réalisation, l'étape de purification par filtration tangentielle comprend au moins une étape d'ultrafiltration et au moins une étape de diafiltration.

Dans un mode de réalisation, l'étape de purification par filtration tangentielle comprend au moins une étape d'ultrafiltration.

Dans un mode de réalisation, l'étape de purification par filtration tangentielle comprend au moins une étape de diafiltration.

Dans un mode de réalisation, la composition de la solution de composé macromoléculaire est modifiée lors de l'étape de purification par filtration tangentielle.

Dans un mode de réalisation, la composition de la solution de composé macromoléculaire est concentrée lors de l'étape de purification par filtration tangentielle.

Dans un mode de réalisation, le procédé de préparation des compositions selon l'invention est caractérisé en ce qu'après l'étape e), on effectue une étape de purification par filtration sur au moins une membrane à fibres creuses.

Dans un mode de réalisation, le procédé de préparation des compositions selon l'invention est caractérisé en ce qu'après l'étape e), on effectue une étape de purification du composé macromoléculaire par précipitation sous forme salifiée.

Dans un mode de réalisation, le procédé de préparation des compositions selon l'invention est caractérisé en ce qu'après l'étape e), on effectue une étape de purification du composé macromoléculaire par précipitation à partir d'une solution obtenue après ajout de sel PBS ou NaCl.

Dans un mode de réalisation, l'étape de purification du composé macromoléculaire par précipitation est effectuée par ajout d'au moins un solvant organique à la solution de composé macromoléculaire.

Dans un mode de réalisation, l'au moins un solvant organique ajouté à la solution de composé macromoléculaire dans l'étape de purification par précipitation est choisi dans le groupe des alcools.

Dans un mode de réalisation, l'éthanol est ajouté à la solution de composé macromoléculaire dans l'étape de purification par précipitation.

Dans un mode de réalisation, l'étape de purification du composé macromoléculaire par précipitation a lieu dans un mélange hydroalcoolique.

Dans un mode de réalisation, le composé macromoléculaire précipité est récupéré par filtration.

Dans un mode de réalisation, le composé macromoléculaire précipité et récupéré par filtration est lavé.

Dans un mode de réalisation, le composé macromoléculaire précipité et récupéré par filtration est séché.

Dans un mode de réalisation, le composé macromoléculaire précipité et récupéré par filtration est séché à atmosphère ambiante.

Dans un mode de réalisation, le composé macromoléculaire précipité et récupéré par filtration est séché sous vide.

Le procédé de préparation des compositions selon l'invention comprend en outre au moins une étape de filtration sur membrane de porosité 0,22 $\mu$m, étape g), de la solution aqueuse de composé macromoléculaire issu de l'étape e), cette étape de filtration étant réalisée sur la solution aqueuse de composé macromoléculaire ou sur un échantillon de la solution aqueuse de composé macromoléculaire.

Dans un mode de réalisation, cette étape de filtration sur membrane de porosité 0,22 $\mu$m est l'étape de stérilisation terminale.

Dans un mode de réalisation, cette étape de filtration sur membrane de porosité 0,22 $\mu$m est effectuée une fois.

Dans un mode de réalisation, cette étape de filtration sur membrane de porosité 0,22 $\mu$m est effectuée deux fois.

Dans un mode de réalisation, cette étape de filtration sur membrane de porosité 0,22 $\mu$m est effectuée trois fois.

Dans un mode de réalisation, l'étape de filtration sur membrane de porosité 0,22 $\mu$m est un moyen de contrôle

en cours du procédé ou « in-process control » (IPC) et est effectuée sur un échantillon de la solution aqueuse de composé macromoléculaire obtenue après l'étape e) du procédé.

Dans un mode de réalisation, l'IPC de filtration sur membrane de porosité 0,22 $\mu$m est réalisé sur un échantillon de la solution aqueuse de composé macromoléculaire obtenue après l'étape e) du procédé après une étape de dilution de l'échantillon.

Dans un mode de réalisation, si l'étape e) de rupture des liaisons glycosidiques est effectuée par autoclavage à la vapeur, alors le procédé de préparation de la composition selon l'invention ne comporte pas d'étape de filtration sur membrane de porosité 0,22 $\mu$m de la solution de composé macromoléculaire.

Dans un mode de réalisation, si l'étape e) de rupture des liaisons glycosidiques est effectuée par autoclavage à la vapeur, alors le procédé de préparation de la composition comporte une étape de prélèvement d'échantillon de la solution aqueuse de composé macromoléculaire après l'étape e) et de test de filtrabilité de l'échantillon sur membrane de porosité 0,22 $\mu$m (IPC).

Dans un mode de réalisation, la composition selon l'invention est caractérisée par G' inférieur ou égal à 300 Pa à 1 Hz.

Dans un mode de réalisation, la composition selon l'invention est caractérisée par G' inférieur ou égal à 150 Pa à 1 Hz.

Dans un mode de réalisation, la composition selon l'invention est caractérisée par G' inférieur ou égal à 100 Pa à 1 Hz.

Dans un mode de réalisation, la composition selon l'invention est caractérisée par G' inférieur ou égal à 85 Pa à 1 Hz.

Dans un mode de réalisation, la composition selon l'invention est caractérisée par G' inférieur ou égal à 70 Pa à 1 Hz.

Dans un mode de réalisation, la composition selon l'invention est caractérisée par Tan $\Delta$ (Tn $\delta$) supérieur ou égal à 1.

Dans un mode de réalisation, la composition selon l'invention est caractérisée par Tan $\Delta$ (Tn $\delta$) compris entre 1 et 1,8 ($1 \leq$ Tan $\Delta$ (Tn $\delta$) $\leq 1{,}8$).

Dans un mode de réalisation, la composition selon l'invention est caractérisée par Tan $\Delta$ (Tn $\delta$) compris entre 1 et 1,5 ($1 \leq$ Tan $\Delta$ (Tn $\delta$) $\leq 1{,}5$).

Dans un mode de réalisation, la composition selon l'invention est caractérisée par Tan $\Delta$ (Tn $\delta$) compris entre 1 et 1,3 ($1 \leq$ Tan $\Delta$ (Tn $\delta$) $\leq 1{,}3$).

L'invention concerne également un procédé de préparation d'une formulation comprenant la composition selon l'invention.

Dans un mode de réalisation, le procédé de préparation d'une formulation à partir de la composition de l'invention comprend en outre au moins une étape de dilution ou de mise en solution.

Dans un mode de réalisation, l'étape de dilution ou de mise en solution est effectuée par addition d'eau ou d'une solution aqueuse saline, par exemple une solution de tampon phosphate, par exemple PBS.

Dans un mode de réalisation, le procédé de préparation d'une formulation comprenant la composition selon l'invention comprend en outre au moins une étape de dilution ou de mise en solution pour obtenir une concentration en composé macromoléculaire comprise entre 2 mg/g et 200 mg/g, par rapport à la masse totale de ladite formulation.

Dans un mode de réalisation, le procédé de préparation d'une formulation comprenant la composition selon l'invention comprend en outre au moins une étape de dilution ou de mise en solution pour obtenir une concentration en composé macromoléculaire comprise entre 2 mg/g et 75 mg/g, par rapport à la masse totale de ladite formulation.

Dans un mode de réalisation, le procédé de préparation d'une formulation comprenant la composition selon l'invention comprend en outre au moins une étape de dilution ou de mise en solution pour obtenir une concentration en composé macromoléculaire comprise entre 5 mg/g et 50 mg/g, par rapport à la masse totale de ladite formulation.

Dans un mode de réalisation, le procédé de préparation d'une formulation comprenant la composition selon l'invention comprend en outre au moins une étape de dilution ou de mise en solution pour obtenir une concentration en composé macromoléculaire comprise entre 10 mg/g et 40 mg/g, par rapport à la masse totale de ladite formulation.

Dans un mode de réalisation, le procédé de préparation d'une formulation comprenant la composition selon l'invention comprend en outre au moins une étape de dilution ou de mise en solution pour obtenir une concentration en composé macromoléculaire d'environ 100 mg/g, par rapport à la masse totale de ladite formulation.

Dans un mode de réalisation, le procédé de préparation d'une formulation comprenant la composition selon l'invention comprend en outre au moins une étape de dilution ou de mise en solution pour obtenir une concentration en composé macromoléculaire d'environ 80 mg/g, par rapport à la masse totale de ladite formulation.

Dans un mode de réalisation, le procédé de préparation d'une formulation comprenant la composition selon l'invention comprend en outre au moins une étape de dilution ou de mise en solution pour obtenir une concentration en composé macromoléculaire d'environ 60 mg/g, par rapport à la masse totale de ladite formulation.

Dans un mode de réalisation, le procédé de préparation d'une formulation comprenant la composition selon

l'invention comprend en outre au moins une étape de dilution ou de mise en solution pour obtenir une concentration en composé macromoléculaire d'environ 40 mg/g, par rapport à la masse totale de ladite formulation.

Dans un mode de réalisation, le procédé de préparation d'une formulation comprenant la composition selon l'invention comprend en outre au moins une étape de dilution ou de mise en solution pour obtenir une concentration en composé macromoléculaire d'environ 20 mg/g, par rapport à la masse totale de ladite formulation.

Dans un mode de réalisation, le procédé de préparation d'une formulation comprenant la composition selon l'invention comprend en outre au moins une étape de dilution ou de mise en solution pour obtenir une concentration en composé macromoléculaire d'environ 10 mg/g, par rapport à la masse totale de ladite formulation.

Dans un mode de réalisation, le procédé de préparation des compositions selon l'invention comprend en outre au moins une étape d'ajout d'au moins un actif.

Dans un mode de réalisation, l'au moins un actif est ajouté sous forme de poudre.

Dans un mode de réalisation, l'au moins un actif est ajouté sous forme de solution ou de suspension.

Dans un mode de réalisation, l'au moins un actif est ajouté sous forme de solution ou de suspension, dans un solvant ou une solution choisie dans le groupe constitué de l'eau, des solutions aqueuses salines par exemple une solution de tampon phosphate, par exemple PBS.

Dans un mode de réalisation, l'au moins un actif est ajouté avant l'étape e) du procédé de préparation des compositions selon l'invention.

Dans un mode de réalisation, l'au moins un actif est ajouté après l'étape e) du procédé de préparation des compositions selon l'invention.

Dans un mode de réalisation, l'au moins un actif est ajouté avant l'étape g) du procédé des compositions selon l'invention.

Dans un mode de réalisation, l'au moins un actif est ajouté après l'étape g) du procédé des compositions selon l'invention.

Dans un mode de réalisation, la formulation obtenue par ajout de l'au moins un actif après l'étape g) du procédé des compositions selon l'invention est stérilisée par filtration sur membrane de porosité 0,22 $\mu$m.

Dans un mode de réalisation, le procédé de préparation d'une formulation comprenant au moins une composition selon l'invention comprend en outre au moins une étape d'ajout d'au moins un actif choisi dans le groupe constitué des anesthésiques locaux, des dérivés de vitamine C, des antiinflammatoires, des antioxydants, et de leurs mélanges.

Dans un mode de réalisation, le procédé de préparation d'une formulation comprenant au moins une composition selon l'invention comprend en outre au moins une étape d'ajout d'au moins un anesthésique local.

Dans un mode de réalisation, le procédé de préparation d'une formulation comprenant au moins une composition selon l'invention comprend en outre au moins une étape d'ajout d'au moins un anesthésique local pour obtenir une concentration en anesthésique local comprise entre 0,1 et 5%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local pour obtenir une concentration en anesthésique local comprise entre 0,1 et 4%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local pour obtenir une concentration en anesthésique local comprise entre 0,1 et 2%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local pour obtenir une concentration en anesthésique local comprise entre 0,1 et 1%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local pour obtenir une concentration en anesthésique local comprise entre 0,1 et 0,5%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local pour obtenir une concentration en anesthésique local d'environ 0,3%, par rapport à la masse totale de ladite formulation.

Dans un mode de réalisation, l'anesthésique local est choisi dans le groupe des amino-esters.

Dans un mode de réalisation, l'amino-ester est choisi dans le groupe comprenant la procaïne, la benzocaïne, la chloroprocaïne et la tétracaïne sous forme de base ou de sel, par exemple sous forme de chlorhydrate.

Dans un mode de réalisation, l'anesthésique local est choisi dans le groupe des amino-amides.

Dans un mode de réalisation, l'amino-amide est choisi dans le groupe comprenant la lidocaïne, la mépivacaïne, la prilocaïne, l'articaïne, l'aptocaïne, la bupivacaïne, l'étidocaïne et la ropivacaïne sous forme de base ou de sel, par exemple sous forme de chlorhydrate.

Dans un mode de réalisation, l'anesthésique local est choisi dans le groupe des amino-éthers.

Dans un mode de réalisation, l'amino-éther est choisi dans le groupe comprenant la diamocaïne et la pramocaïne sous forme de base ou de sel, par exemple sous forme de chlorhydrate ou de cyclamate.

Dans un mode de réalisation, l'amino-éther est choisi dans le groupe constitué par la lidocaïne, la mépivacaïne, et leurs sels et leurs isomères isolés.

Dans un mode de réalisation, l'amino-éther est la lidocaïne.

Dans un mode de réalisation, l'amino-éther est la lidocaïne ou l'un de ses sels pharmaceutiquement acceptables.

Dans un mode de réalisation, l'amino-éther est le chlorhydrate de lidocaïne.

Dans un mode de réalisation, l'amino-éther est la mépivacaïne.

Dans un mode de réalisation, l'amino-éther est la mépivacaïne ou l'un de ses sels pharmaceutiquement acceptables.

Dans un mode de réalisation, l'amino-éther est choisi dans le groupe constitué par le chlorhydrate de mépivacaïne racémique, le chlorhydrate de mépivacaïne racémique, le chlorhydrate de (r)-mépivacaïne, le chlorhydrate de (s)-mépivacaïne, la (r)-mépivacaïne et la (s)-mépivacaïne, ou l'un de leurs sels pharmaceutiquement acceptables.

Dans un mode de réalisation, l'amino-éther est le chlorhydrate de mépivacaïne.

Dans un mode de réalisation, l'amino-éther est le chlorhydrate de (r)-mépivacaïne.

Dans un mode de réalisation, l'amino-éther est le chlorhydrate de (s)-mépivacaïne.

Dans un mode de réalisation, l'amino-éther est le chlorhydrate de mépivacaïne racémique.

Dans un mode de réalisation, l'amino-éther est la (r)-mépivacaïne.

Dans un mode de réalisation, l'amino-éther est la (s)-mépivacaïne.

Dans un mode de réalisation, l'amino-éther est la mépivacaïne racémique.

Dans un mode de réalisation, le procédé de préparation d'une formulation comprenant au moins une composition selon l'invention comprend en outre au moins une étape d'ajout d'au moins un anesthésique local amino-éther choisi dans le groupe constitué de la lidocaïne, de la mépivacaïne, et de leurs mélanges.

Dans un mode de réalisation, le procédé de préparation d'une formulation comprenant au moins une composition selon l'invention comprend en outre au moins une étape d'ajout d'au moins un anesthésique local étant la lidocaïne.

Dans un mode de réalisation, le procédé de préparation d'une formulation comprenant au moins une composition selon l'invention comprend en outre au moins une étape d'ajout d'au moins un anesthésique local étant la lidocaïne pour obtenir une concentration en lidocaïne comprise entre 0,1 et 5%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la lidocaïne pour obtenir une concentration en lidocaïne comprise entre 0,1 et 4%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la lidocaïne pour obtenir une concentration en lidocaïne comprise entre 0,1 et 2%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la lidocaïne pour obtenir une concentration en lidocaïne comprise entre 0,1 et 1%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la lidocaïne pour obtenir une concentration en lidocaïne comprise entre 0,1 et 0,5%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la lidocaïne pour obtenir une concentration en anesthésique local d'environ 0,3%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la mépivacaïne.

Dans un mode de réalisation, le procédé de préparation d'une formulation comprenant au moins une composition selon l'invention comprend en outre au moins une étape d'ajout d'au moins un anesthésique local étant la mépivacaïne pour obtenir une concentration en mépivacaïne comprise entre 0,1 et 5%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la mépivacaïne pour obtenir une concentration en mépivacaïne comprise entre 0,1 et 4%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la mépivacaïne pour obtenir une concentration en mépivacaïne comprise entre 0,1 et 2%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la mépivacaïne pour obtenir une concentration en mépivacaïne comprise entre 0,1 et 1%, par rapport à la masse totale de ladite

formulation.

Dans un mode de réalisation, le procédé de préparation d'une formulation comprenant au moins une composition selon l'invention comprend en outre au moins une étape d'ajout d'au moins un anesthésique local étant la mépivacaïne pour obtenir une concentration en mépivacaïne comprise entre 0,1 et 0,5%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la mépivacaïne pour obtenir une concentration en anesthésique local d'environ 0,3%, par rapport à la masse totale de ladite formulation.

Dans un mode de réalisation, le procédé de préparation d'une formulation comprenant au moins une composition selon l'invention comprend en outre au moins une étape d'ajout d'au moins un anesthésique local étant la dyclonine sous forme de base ou de sel, par exemple sous forme de chlorhydrate.

Dans un mode de réalisation, le procédé de préparation d'une formulation comprenant au moins une composition selon l'invention comprend en outre au moins une étape d'ajout d'au moins un anesthésique local choisi dans le groupe constitué par le chlorobutanol, le guafécaïnol et le polidocanol.

Dans un mode de réalisation, l'anesthésique local est le chlorobutanol.

Dans un mode de réalisation, l'anesthésique local est le guafécaïnol.

Dans un mode de réalisation, l'anesthésique local est le polidocanol.

Dans un mode de réalisation, le procédé de préparation d'une formulation comprenant au moins une composition selon l'invention comprend en outre au moins une étape d'ajout d'au moins un antiinflammatoire.

Dans un mode de réalisation, le procédé de préparation d'une formulation comprenant au moins une composition selon l'invention comprend en outre au moins une étape d'ajout d'au moins un antiinflammatoire choisi dans le groupe constitué des antiinflammatoires stéroïdiens et non stéroïdiens.

Dans un mode de réalisation, le procédé de préparation d'une formulation comprenant au moins une composition selon l'invention comprend en outre au moins une étape d'ajout d'au moins un antiinflammatoire choisi dans le groupe constitué des antiinflammatoires non stéroïdiens.

Dans un mode de réalisation, le procédé de préparation d'une formulation comprenant au moins une composition selon l'invention comprend en outre au moins une étape d'ajout d'au moins un antiinflammatoire choisi dans le groupe comprenant les antiinflammatoires salicylés, les dérivés propioniques, les dérivés indoliques, les dérivés pyrazolés, les oxicams et les coxibs.

Dans un mode de réalisation, le procédé de préparation d'une formulation comprenant au moins une composition selon l'invention comprend en outre au moins une étape d'ajout d'au moins un antiinflammatoire choisi dans le groupe comprenant le diclofénac, le nimésulfide, l'acide niflumique, l'acide méfénamique et la nabumétone, seuls ou en mélange.

Dans un mode de réalisation, le procédé de préparation d'une formulation comprenant au moins une composition selon l'invention comprend en outre au moins une étape d'ajout d'au moins un antiinflammatoire salicylé choisi dans le groupe comprenant le diflunisal, le bénorilate et l'aspirine, seuls ou en mélange.

Dans un mode de réalisation, le procédé de préparation d'une formulation comprenant au moins une composition selon l'invention comprend en outre au moins une étape d'ajout d'au moins un antiinflammatoire choisi dans le groupe des dérivés propioniques comprenant l'alminoprofène, le kétoprofène, l'ibuprofène, le naproxène, le flurbiprofène et l'acide tiaprofènique, seuls ou en mélange.

Dans un mode de réalisation, le procédé de préparation d'une formulation comprenant au moins une composition selon l'invention comprend en outre au moins une étape d'ajout d'au moins un antiinflammatoire choisi dans le groupe des dérivés indoliques comprenant l'indométacine, le sulindac et l'étodolac, seuls ou en mélange.

Dans un mode de réalisation, le procédé de préparation d'une formulation comprenant au moins une composition selon l'invention comprend en outre au moins une étape d'ajout d'au moins un antiinflammatoire choisi dans le groupe des dérivés pyrazolés comprenant notamment la phénylbutazone.

Dans un mode de réalisation, le procédé de préparation d'une formulation comprenant au moins une composition selon l'invention comprend en outre au moins une étape d'ajout d'au moins un antiinflammatoire choisi dans le groupe des oxicams comprenant le piroxicam, le ténoxicam et le méloxicam, seuls ou en mélange.

Dans un mode de réalisation, le procédé de préparation d'une formulation comprenant au moins une composition selon l'invention comprend en outre au moins une étape d'ajout d'au moins un antiinflammatoire choisi dans le groupe des coxibs comprenant le célécoxib, l'étoricoxib et le rofécoxib, seuls ou en mélange.

Dans un mode de réalisation, la concentration en antiinflammatoires non stéroïdiens dans une formulation comprenant au moins une composition selon l'invention est comprise entre 0,01 et 2000 mg/g.

Dans un mode de réalisation, la concentration en antiinflammatoires non stéroïdiens dans une formulation comprenant au moins une composition selon l'invention est comprise entre 0,1 et 1000 mg/g.

Dans un mode de réalisation, la concentration en antiinflammatoires non stéroïdiens dans une formulation comprenant au moins une composition selon l'invention est comprise entre 0,5 et 500 mg/g.

Dans un mode de réalisation, le procédé de préparation d'une formulation comprenant au moins une composition

selon l'invention comprend en outre au moins une étape d'ajout d'au moins un antiinflammatoire stéroïdien.

Dans un mode de réalisation, le procédé de préparation d'une formulation comprenant au moins une composition selon l'invention comprend en outre au moins une étape d'ajout d'au moins un antiinflammatoire stéroïdien choisi dans le groupe comprenant la dexaméthasone, la prednisolone, la corticostérone, le budésonide, la sulfasalazine, la mésalamine, la cétirizine, la diphénhydramine, l'antipyrine, le salicylate de méthyle, la loratadine, le thymol, le carvacrol, le bisabolol, l'allantoïne, l'eucalyptol, la phénazone (antipyrine), la propyphénazone, seuls ou en mélange.

Dans un mode de réalisation, la concentration en antiinflammatoires stéroïdiens dans une formulation comprenant au moins une composition selon l'invention est comprise entre 0,01 et 2000 mg/g.

Dans un mode de réalisation, la concentration en antiinflammatoires stéroïdiens dans une formulation comprenant au moins une composition selon l'invention est comprise entre 0,1 et 1000 mg/g.

Dans un mode de réalisation, la concentration en antiinflammatoires stéroïdiens dans une formulation comprenant au moins une composition selon l'invention est comprise entre 0,5 et 500 mg/g.

Dans un mode de réalisation, le procédé de préparation d'une formulation comprenant au moins une composition selon l'invention comprend en outre au moins une étape d'ajout d'au moins un antiinflammatoire choisi dans le groupe constitué du sucrose octasulfate et de ses sels.

Dans un mode de réalisation, le procédé de préparation d'une formulation comprenant au moins une composition selon l'invention comprend en outre au moins une étape d'ajout d'au moins un antiinflammatoire choisi dans le groupe constitué du sucrose octasulfate et de ses sels de sodium et de potassium.

Dans un mode de réalisation, le procédé de préparation d'une formulation comprenant au moins une composition selon l'invention comprend en outre au moins une étape d'ajout d'au moins un antiinflammatoire sel hydrosoluble de sucrose octasulfate choisi dans le groupe constitué par les sels de métaux alcalins, les sels de métaux alcalino-terreux, les sels d'argent, les sels d'ammonium, les sels d'acides aminés.

Dans un mode de réalisation, le procédé de préparation d'une formulation comprenant au moins une composition selon l'invention comprend en outre au moins une étape d'ajout d'au moins un antiinflammatoire sel hydrosoluble de sucrose octasulfate choisi dans le groupe constitué par les sels de métaux alcalins ou les sels de métaux alcalino-terreux.

Dans un mode de réalisation, le procédé de préparation d'une formulation comprenant au moins une composition selon l'invention comprend en outre au moins une étape d'ajout d'au moins un antiinflammatoire sel hydrosoluble de sucrose octasulfate choisi dans le groupe constitué par le sel de sodium de sucrose octasulfate ou le sel de potassium de sucrose octasulfate.

Dans un mode de réalisation, le procédé de préparation d'une formulation comprenant au moins une composition selon l'invention comprend en outre au moins une étape d'ajout d'au moins un antimicrobien.

Dans un mode de réalisation, le procédé de préparation d'une formulation comprenant au moins une composition selon l'invention comprend en outre au moins une étape d'ajout d'au moins un antimicrobien choisi dans le groupe comprenant la gentamicine, la sulfadiazine d'argent, le métronidazole, la fucidine, la bacitracine, l'éosine, la povidone iodée, le gluconate de cuivre, le gluconate de zinc, le gluconate de manganèse ou leur sels, seuls ou en mélange.

Dans un mode de réalisation, la concentration en antimicrobiens dans une formulation comprenant au moins une composition selon l'invention est comprise entre 0,1 et 200 mg/g.

Dans un mode de réalisation, la concentration en antimicrobiens dans une formulation comprenant au moins une composition selon l'invention est comprise entre 0,5 et 100 mg/g.

Dans un mode de réalisation, le procédé de préparation d'une formulation comprenant au moins une composition selon l'invention comprend en outre au moins une étape d'ajout d'au moins un glycoside ou un dérivé de glycoside.

Dans un mode de réalisation, le procédé de préparation d'une formulation comprenant au moins une composition selon l'invention comprend en outre au moins une étape d'ajout d'au moins un glycoside ou un dérivé de glycoside choisi dans le groupe comprenant le D-glucopyranose, le 1,4 glycoside, l'esculine, l'hespéridine, la diosmine, l'arbutine, la skimmine ou l'aloïne, seuls ou en mélanges.

Dans un mode de réalisation, la concentration en glycosides dans une formulation comprenant au moins une composition selon l'invention est comprise entre 0,1 et 200 mg/g.

Dans un mode de réalisation, la concentration en glycosides dans une formulation comprenant au moins une composition selon l'invention est comprise entre 0,5 et 100 mg/g.

Dans un mode de réalisation, le procédé de préparation d'une formulation comprenant au moins une composition selon l'invention comprend en outre au moins une étape d'ajout d'au moins un hydratant ou régénérant tissulaire.

Dans un mode de réalisation, le procédé de préparation d'une formulation comprenant au moins une composition selon l'invention comprend en outre au moins une étape d'ajout d'au moins un hydratant ou régénérant tissulaire choisi dans le groupe constitué des macro-éléments.

Dans un mode de réalisation, le procédé de préparation d'une formulation comprenant au moins une composition selon l'invention comprend en outre au moins une étape d'ajout d'au moins un macro-élément choisi dans le groupe comprenant les gluconates de fer, calcium, cuivre, zinc, manganèse, magnésium ou potassium, du timéthylsilanol, du triméthylsilanolate, du triméthylsilanolate de potassium, du méthylsilanol mannuronate, du monoéthyltrisilanol orthohydroxybenzoate de sodium, seuls ou en mélange.

Dans un mode de réalisation, le procédé de préparation d'une formulation comprenant au moins une composition selon l'invention comprend en outre au moins une étape d'ajout d'au moins un hydratant ou régénérant tissulaire choisi dans le groupe constitué des acides aminés essentiels.

Dans un mode de réalisation, le procédé de préparation d'une formulation comprenant au moins une composition selon l'invention comprend en outre au moins une étape d'ajout d'au moins acide aminé essentiel choisi dans le groupe comprenant l'isoleucine, la leucine, la lysine, la méthionine, la phénylalanine, la thréonine, le tryptophane et la valine, seuls ou en mélange.

Dans un mode de réalisation, le procédé de préparation d'une formulation comprenant au moins une composition selon l'invention comprend en outre au moins une étape d'ajout d'au moins un hydratant ou régénérant tissulaire choisi dans le groupe constitué des acides aminés semi-essentiels.

Dans un mode de réalisation, le procédé de préparation d'une formulation comprenant au moins une composition selon l'invention comprend en outre au moins une étape d'ajout d'au moins un acide aminé semi-essentiel choisi dans le groupe comprenant l'arginine et l'histidine, seuls ou en mélange.

Dans un mode de réalisation, le procédé de préparation d'une formulation comprenant au moins une composition selon l'invention comprend en outre au moins une étape d'ajout d'au moins un hydratant ou régénérant tissulaire choisi dans le groupe constitué des acides aminés non essentiels.

Dans un mode de réalisation, le procédé de préparation d'une formulation comprenant au moins une composition selon l'invention comprend en outre au moins une étape d'ajout d'au moins un acide aminé non essentiel choisi dans le groupe comprenant l'alanine, l'asparagine, l'acide aspartique, la cystéine, la glutamine, l'acide glutamique, la glycine, la proline, la sérine, la tyrosine, seuls ou en mélange.

Dans un mode de réalisation, le procédé de préparation d'une formulation comprenant au moins une composition selon l'invention comprend en outre au moins une étape d'ajout d'au moins un hydratant ou régénérant tissulaire choisi dans le groupe constitué des vitamines.

Dans un mode de réalisation, le procédé de préparation d'une formulation comprenant au moins une composition selon l'invention comprend en outre au moins une étape d'ajout d'au moins une vitamine choisie dans le groupe constitué du rétinol, de la thiamine, la riboflavine, du nicotinamide, de l'adénine, du pantothénate de calcium, de la pyridoxine, de l'inositol, de la biotine, de l'acide folique, de l'acides para-amino-benzoïque, de la cobalamine, de la vitamine C, du chlorure de choline, seuls ou en mélange.

Dans un mode de réalisation, le procédé de préparation d'une formulation comprenant au moins une composition selon l'invention comprend en outre au moins une étape d'ajout d'au moins un hydratant ou régénérant tissulaire choisi dans le groupe constitué des acides nucléiques.

Dans un mode de réalisation, le procédé de préparation d'une formulation comprenant au moins une composition selon l'invention comprend en outre au moins une étape d'ajout d'au moins un acide nucléique choisi dans le groupe constitué de la déoxyadénosine, de la déoxycytidine, de la déoxyguanosine, de la déoxythymidine, de la méthylcytosine, seuls ou en mélange.

Dans un mode de réalisation, le procédé de préparation d'une formulation comprenant au moins une composition selon l'invention comprend en outre au moins une étape d'ajout d'au moins un hydratant ou régénérant tissulaire choisi dans le groupe constitué des coenzymes.

Dans un mode de réalisation, le procédé de préparation d'une formulation comprenant au moins une composition selon l'invention comprend en outre au moins une étape d'ajout d'au moins une coenzyme choisie dans le groupe constitué de la thiamine pyrophosphate, du coenzyme A, du FAD, du NAD, du NADP, de l'UTP, seuls ou en mélange.

Dans un mode de réalisation, le procédé de préparation d'une formulation comprenant au moins une composition selon l'invention comprend en outre au moins une étape d'ajout d'au moins un hydratant ou régénérant tissulaire choisi dans le groupe constitué de la déoxythymidine, du glutathion, du pyruvate de sodium, de l'acide lipoïque et de la putrescine, seuls ou en mélange.

Dans un mode de réalisation, la concentration en hydratants ou régénérants tissulaires dans une formulation comprenant au moins une composition selon l'invention est comprise entre 0,01 et 500 mg/g.

Dans un mode de réalisation, la concentration en hydratants ou régénérants tissulaires dans une formulation comprenant au moins une composition selon l'invention est comprise entre 0,1 et 200 mg/g.

Dans un mode de réalisation, le procédé de préparation d'une formulation comprenant au moins une composition selon l'invention comprend en outre au moins une étape d'ajout d'au moins un antioxydant.

Dans un mode de réalisation, le procédé de préparation d'une formulation comprenant au moins une composition selon l'invention comprend en outre au moins une étape d'ajout d'au moins un antioxydant choisi dans le groupe des polyols.

Dans un mode de réalisation, le procédé de préparation d'une formulation comprenant au moins une composition selon l'invention comprend en outre au moins une étape d'ajout d'au moins un polyol choisi dans le groupe constitué du mannitol, du sorbitol, du propylène glycol, du xylitol, du glycérol, du maltitol, du lactitol et de l'érythritol.

Dans un mode de réalisation, le procédé de préparation d'une formulation comprenant au moins une composition selon l'invention comprend en outre au moins une étape d'ajout d'au moins un polyol choisi dans le groupe constitué du mannitol, du sorbitol, du maltitol et du glycérol, seul ou en mélange.

Dans un mode de réalisation, le procédé de préparation d'une formulation comprenant au moins une composition selon l'invention comprend en outre au moins une étape d'ajout d'au moins un polyol choisi dans le groupe constitué du mannitol, du sorbitol et du maltitol, seul ou en mélange.

Dans un mode de réalisation, le procédé de préparation d'une formulation comprenant au moins une composition selon l'invention comprend en outre au moins une étape d'ajout d'au moins un polyol pour obtenir une concentration en polyol comprise entre 0,1 mg/ml et 50 mg/ml, par rapport à la masse totale de ladite formulation.

Dans un mode de réalisation, le procédé de préparation d'une formulation comprenant au moins une composition selon l'invention comprend en outre au moins une étape d'ajout d'au moins un polyol pour obtenir une concentration en polyol comprise entre 5 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout d'au moins un polyol pour obtenir une concentration en polyol comprise entre 10 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout d'au moins un polyol pour obtenir une concentration en polyol comprise entre 20 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout d'au moins un polyol pour obtenir une concentration en polyol comprise entre 30 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout d'au moins un polyol étant le mannitol.

Dans un mode de réalisation, le procédé de préparation d'une formulation comprenant au moins une composition selon l'invention comprend en outre au moins une étape d'ajout de mannitol pour obtenir une concentration en mannitol comprise entre 5 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout de mannitol pour obtenir une concentration en mannitol comprise entre 10 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout de mannitol pour obtenir une concentration en mannitol comprise entre 20 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout de mannitol pour obtenir une concentration en mannitol comprise entre 30 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout d'au moins un polyol étant le sorbitol.

Dans un mode de réalisation, le procédé de préparation d'une formulation comprenant au moins une composition selon l'invention comprend en outre au moins une étape d'ajout de sorbitol pour obtenir une concentration en sorbitol comprise entre 5 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout de sorbitol pour obtenir une concentration en sorbitol comprise entre 10 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout de sorbitol pour obtenir une concentration en sorbitol comprise entre 20 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout de sorbitol pour obtenir une concentration en sorbitol comprise entre 30 mg/ml et 40 mg/ml, par rapport à la masse totale de ladite formulation.

Dans un mode de réalisation, le procédé de préparation d'une formulation comprenant au moins une composition selon l'invention comprend en outre au moins une étape d'ajout d'au moins un polyol étant le maltitol.

Dans un mode de réalisation, le procédé de préparation d'une formulation comprenant au moins une composition selon l'invention comprend en outre au moins une étape d'ajout d'au moins un polyol étant le glycérol.

Dans un mode de réalisation, le procédé de préparation d'une formulation comprenant au moins une composition selon l'invention comprend en outre au moins une étape d'ajout d'un mélange de mannitol et de sorbitol.

Dans un mode de réalisation, le procédé de préparation d'une formulation comprenant au moins une composition selon l'invention comprend en outre au moins une étape d'ajout d'au moins un antioxydant choisi dans le groupe des dérivés de vitamine C.

Dans un mode de réalisation, le procédé de préparation d'une formulation comprenant au moins une composition selon l'invention comprend en outre au moins une étape d'ajout d'au moins un antioxydant choisi dans le groupe des dérivés de vitamine C comprenant le phosphate d'ascorbyle de magnésium, le phosphate d'ascorbyle de sodium, l'ascorbyl-2-glucoside, et de leur mélange.

Dans un mode de réalisation, ledit au moins un dérivé de vitamine C est le phosphate d'ascorbyle de magnésium.

Dans un mode de réalisation, le procédé de préparation d'une formulation comprenant au moins une composition selon l'invention comprend en outre au moins une étape d'ajout d'au moins un antioxydant choisi dans le groupe des dérivés de vitamine E et des tocophérols.

Dans un mode de réalisation, le procédé de préparation d'une formulation comprenant au moins une composition selon l'invention comprend en outre au moins une étape d'ajout d'au moins un antioxydant choisi dans le groupe des caroténoïdes et des rétinoïdes et de leurs dérivés.

Dans un mode de réalisation, le procédé de préparation d'une formulation comprenant au moins une composition selon l'invention comprend en outre au moins une étape d'ajout d'au moins un antioxydant choisi dans le groupe des caroténoïdes et des rétinoïdes et de leurs dérivés comprenant le rétinol, l'acide rétinoïque le rétinal, les esters de rétinol et le carotène.

Dans un mode de réalisation, le procédé de préparation d'une formulation comprenant au moins une composition selon l'invention comprend en outre au moins une étape d'ajout d'au moins un antioxydant choisi dans le groupe des pseudo-tripeptides.

Dans un mode de réalisation, le pseudo-tripeptide est le glutathion.

Dans un mode de réalisation, le procédé de préparation d'une formulation comprenant au moins une composition selon l'invention comprend en outre au moins une étape d'ajout d'au moins un antioxydant choisi dans le groupe comprenant les différentes formes de la coenzyme Q10, l'ubiquinone ou l'ubiquinol.

Dans un mode de réalisation, le procédé de préparation d'une formulation comprenant au moins une composition selon l'invention comprend en outre au moins une étape d'ajout d'au moins une vitamine.

Dans un mode de réalisation, le procédé de préparation d'une formulation comprenant au moins une composition selon l'invention comprend en outre au moins une étape d'ajout d'au moins une vitamine choisie dans le groupe comprenant le rétinol, la thiamine, la riboflavine, le nicotinamide, le dexpenthénol, la piridoxine, l'acide ascorbique, l'ergocalciférol, le tocophérol, la biotine et l'acide folique seuls ou en mélange.

Dans un mode de réalisation, la concentration en vitamines dans une formulation comprenant au moins une composition selon l'invention est comprise entre 0,01 et 200 mg/g.

Dans un mode de réalisation, la concentration en vitamines dans une formulation comprenant au moins une composition selon l'invention est comprise entre 0,01 et 100 mg/g.

Dans un mode de réalisation, la concentration en vitamines dans une formulation comprenant au moins une composition selon l'invention est comprise entre 0,5 et 50 mg/g.

Dans un mode de réalisation, le procédé de préparation d'une formulation comprenant au moins une composition selon l'invention comprend en outre au moins une étape d'ajout d'au moins un acide aminé.

Dans un mode de réalisation, le procédé de préparation d'une formulation comprenant au moins une composition selon l'invention comprend en outre au moins une étape d'ajout d'au moins un acide aminé choisi dans le groupe constitué des acides aminés essentiels, acides aminés semi-essentiels et acides aminés non essentiels, seuls ou en mélange.

Dans un mode de réalisation, le procédé de préparation d'une formulation comprenant au moins une composition selon l'invention comprend en outre au moins une étape d'ajout d'au moins un acide aminé choisi dans le groupe des acides aminés essentiels.

Dans un mode de réalisation, le procédé de préparation d'une formulation comprenant au moins une composition selon l'invention comprend en outre au moins une étape d'ajout d'au moins un acide aminé essentiel choisi dans le groupe comprenant l'isoleucine, la leucine, la lysine, la méthionine, la phénylalanine, la thréonine, le tryptophane et la valine, seuls ou en mélange.

Dans un mode de réalisation, le procédé de préparation d'une formulation comprenant au moins une composition selon l'invention comprend en outre au moins une étape d'ajout d'au moins un acide aminé choisi dans le groupe des acides aminés semi-essentiels.

Dans un mode de réalisation, le procédé de préparation d'une formulation comprenant au moins une composition selon l'invention comprend en outre au moins une étape d'ajout d'au moins un acide aminé semi-essentiel choisi dans le groupe comprenant l'arginine et l'histidine, seuls ou en mélange.

Dans un mode de réalisation, le procédé de préparation d'une formulation comprenant au moins une composition selon l'invention comprend en outre au moins une étape d'ajout d'au moins un acide aminé choisi dans le groupe des acides aminés non essentiels.

Dans un mode de réalisation, le procédé de préparation d'une formulation comprenant au moins une composition selon l'invention comprend en outre au moins une étape d'ajout d'au moins un acide aminé non essentiel choisi dans

le groupe comprenant l'alanine, l'asparagine, l'acide aspartique, la cystéine, la glutamine, l'acide glutamique, la glycine, la proline, la sérine, la tyrosine, seuls ou en mélange.

Dans un mode de réalisation, le procédé de préparation d'une formulation comprenant au moins une composition selon l'invention comprend en outre au moins une étape d'ajout d'au moins un acide aminé choisi dans le groupe comprenant l'hydroxyproline, la taurine et l'ornithine, seuls ou en mélange.

Dans un mode de réalisation, la concentration en acides aminés dans une formulation comprenant au moins une composition selon l'invention est comprise entre 0,01 et 150 mg/g.

Dans un mode de réalisation, la concentration en acides aminés dans une formulation comprenant au moins une composition selon l'invention est comprise entre 0,01 et 100 mg/g.

Dans un mode de réalisation, la concentration en acides aminés dans une formulation comprenant au moins une composition selon l'invention est comprise entre 0,5 et 50 mg/g.

Dans un mode de réalisation, le procédé de préparation d'une formulation comprenant au moins une composition selon l'invention comprend en outre au moins une étape d'ajout d'au moins un vasoconstricteur.

Dans un mode de réalisation, le procédé de préparation d'une formulation comprenant au moins une composition selon l'invention comprend en outre au moins une étape d'ajout d'au moins un vasoconstricteur choisi dans le groupe comprenant la naphazoline, l'épinéphrine, la méthoxamine, la méthylnorépinéphrine, la norépinéphrine, l'oxyméthazoline, la phényléphrine, la pseudoéphédrine, la synéphrine, la cirazoline et la xylométhazoline.

Dans un mode de réalisation, la concentration en vasoconstricteurs dans une formulation comprenant au moins une composition selon l'invention est comprise entre 0,01 et 3 mg/g.

Dans un mode de réalisation, le procédé de préparation d'une formulation comprenant au moins une composition selon l'invention comprend en outre au moins une étape d'ajout d'au moins un vasodilatateur.

Dans un mode de réalisation, le procédé de préparation d'une formulation comprenant au moins une composition selon l'invention comprend en outre au moins une étape d'ajout d'au moins un vasodilatateur choisi dans le groupe comprenant l'adénosine, l'acide nicotinique, le minoxidil et le diazoxide, seuls ou en mélange.

Dans un mode de réalisation, la concentration en vasodilatateurs dans une formulation comprenant au moins une composition selon l'invention est comprise entre 0,01 et 10 mg/g.

Dans un mode de réalisation, le procédé de préparation d'une formulation comprenant au moins une composition selon l'invention comprend en outre au moins une étape d'ajout d'au moins un anti-hémorragique ou hémostatique.

Dans un mode de réalisation, le procédé de préparation d'une formulation comprenant au moins une composition selon l'invention comprend en outre au moins une étape d'ajout d'au moins un anti-hémorragique ou hémostatique choisi dans le groupe comprenant l'acide aminocaproïque ou l'acide tranexamique, seuls ou en mélange.

Dans un mode de réalisation, la concentration en anti-hémorragiques ou hémostatiques dans une formulation comprenant au moins une composition selon l'invention est comprise entre 0,01 et 5 mg/g.

Dans un mode de réalisation, le procédé de préparation d'une formulation comprenant au moins une composition selon l'invention comprend en outre au moins une étape d'ajout d'au moins un antioxydant et au moins un anesthésique local.

Dans un mode de réalisation, le procédé de préparation d'une formulation comprenant au moins une composition selon l'invention comprend en outre au moins une étape d'ajout d'au moins un antioxydant choisi dans le groupe des polyols et au moins un anesthésique local choisi dans le groupe des amino-amides.

Dans un mode de réalisation, le procédé de préparation d'une formulation comprenant au moins une composition selon l'invention comprend en outre au moins une étape d'ajout d'au moins un antioxydant, au moins une vitamine et au moins un régénérant tissulaire.

Dans un mode de réalisation, le procédé de préparation d'une formulation comprenant au moins une composition selon l'invention comprend en outre au moins une étape d'ajout d'au moins un acide aminé, au moins une vitamine et au moins un régénérant tissulaire.

Dans un mode de réalisation, le procédé de préparation d'une formulation comprenant au moins une composition selon l'invention comprend en outre au moins une étape d'ajout d'au moins un antioxydant, au moins un acide aminé, au moins une vitamine et au moins un régénérant tissulaire.

Dans un mode de réalisation, le procédé de préparation d'une formulation comprenant au moins une composition selon l'invention comprend en outre au moins une étape d'ajout d'au moins un actif choisi dans le groupe constitué des actifs thermosensibles.

Dans un mode de réalisation, l'au moins un actif thermosensible est ajouté après l'étape e) du procédé de préparation de l'au moins une composition selon l'invention.

Dans un mode de réalisation, le procédé de préparation d'une formulation comprenant au moins une composition selon l'invention comprend en outre au moins une étape d'ajout d'un actif dit thermosensible d'origine naturelle.

Dans un mode de réalisation, le procédé de préparation d'une formulation comprenant au moins une composition selon l'invention comprend en outre au moins une étape d'ajout d'un actif dit thermosensible d'origine synthétique.

Dans un mode de réalisation, le procédé de préparation d'une formulation comprenant au moins une composition

selon l’invention comprend en outre au moins une étape d’ajout d’au moins un actif thermosensible choisi dans le groupe constitué des peptides, des hormones, des protéines, des facteurs de croissance, des anticorps et des vitamines, seuls ou en mélange.

Dans un mode de réalisation, le procédé de préparation d’une formulation comprenant au moins une composition selon l’invention comprend en outre au moins une étape d’ajout d’au moins un actif thermosensible choisi dans le groupe constitué des peptides.

Dans un mode de réalisation, l’actif thermosensible ajouté est un peptide.

Dans un mode de réalisation, le procédé de préparation d’une formulation comprenant au moins une composition selon l’invention comprend en outre au moins une étape d’ajout d’au moins un actif thermosensible choisi dans le groupe constitué des hormones.

Dans un mode de réalisation, l’actif thermosensible ajouté est une hormone.

Dans un mode de réalisation, le procédé de préparation d’une formulation comprenant au moins une composition selon l’invention comprend en outre au moins une étape d’ajout d’au moins un actif thermosensible choisi dans le groupe constitué des protéines.

Dans un mode de réalisation, l’actif thermosensible ajouté est une protéine.

Dans un mode de réalisation, le procédé de préparation d’une formulation comprenant au moins une composition selon l’invention comprend en outre au moins une étape d’ajout d’au moins un actif thermosensible choisi dans le groupe constitué facteurs de croissance.

Dans un mode de réalisation, l’actif thermosensible ajouté est un facteur de croissance.

Dans un mode de réalisation, le procédé de préparation d’une formulation comprenant au moins une composition selon l’invention comprend en outre au moins une étape d’ajout d’au moins un actif thermosensible choisi dans le groupe constitué des anticorps.

Dans un mode de réalisation, l’actif thermosensible ajouté est un anticorps.

Dans un mode de réalisation, le procédé de préparation d’une formulation comprenant au moins une composition selon l’invention comprend en outre au moins une étape d’ajout d’au moins un actif thermosensible choisi dans le groupe constitué des vitamines.

Dans un mode de réalisation, les vitamines sont choisies dans le groupe comprenant le rétinol, la thiamine, la riboflavine, le nicotinamide, le dexpenthénol, la piridoxine, l’acide ascorbique, l’ergocalciférol, le tocophérol, la biotine et l’acide folique seuls ou en mélange.

L’invention concerne également une formulation comprenant au moins une composition selon l’invention.

Dans un mode de réalisation, la formulation est caractérisée en ce que la concentration en composé macromoléculaire est comprise entre 2 mg/g et 100 mg/g, par rapport à la masse totale de ladite formulation.

Dans un mode de réalisation, la formulation est caractérisée en ce que la concentration en composé macromoléculaire est comprise entre 2 mg/g et 75 mg/g, par rapport à la masse totale de ladite formulation.

Dans un mode de réalisation, la formulation est caractérisée en ce que la concentration en composé macromoléculaire est comprise entre 5 mg/g et 50 mg/g, par rapport à la masse totale de ladite formulation.

Dans un mode de réalisation, la formulation est caractérisée en ce que la concentration en composé macromoléculaire est comprise entre 10 mg/g et 40 mg/g, par rapport à la masse totale de ladite formulation.

Dans un mode de réalisation, la formulation est caractérisée en ce que la concentration en composé macromoléculaire est d’environ 100 mg/g, par rapport à la masse totale de ladite formulation.

Dans un mode de réalisation, la formulation est caractérisée en ce que la concentration en composé macromoléculaire est d’environ 80 mg/g, par rapport à la masse totale de ladite formulation.

Dans un mode de réalisation, la formulation est caractérisée en ce que la concentration en composé macromoléculaire est d’environ 60 mg/g, par rapport à la masse totale de ladite formulation.

Dans un mode de réalisation, la formulation est caractérisée en ce que la concentration en composé macromoléculaire est d’environ 40 mg/g, par rapport à la masse totale de ladite formulation.

Dans un mode de réalisation, la formulation est caractérisée en ce que la concentration en composé macromoléculaire est d’environ 20 mg/g, par rapport à la masse totale de ladite formulation.

Dans un mode de réalisation, la formulation est caractérisée en ce que la concentration en composé macromoléculaire est d’environ 10 mg/g, par rapport à la masse totale de ladite formulation.

Dans un mode de réalisation, la formulation est caractérisée en ce qu’elle est injectable.

Dans un mode de réalisation, la formulation est caractérisée en ce qu’elle est stérile.

Dans un mode de réalisation, la formulation est caractérisée en ce qu’elle est injectable et stérile.

Les formulations obtenues à base de compositions selon l’invention ont de nombreuses applications.

Parmi les applications médicales, on citera par exemple les injections pour remplacer les liquides biologiques déficients, par exemple dans les articulations pour remplacer le liquide synovial, l’injection par suite d’une chirurgie pour éviter les adhérences post-chirurgicales, les injections périurétrales pour traiter l’incontinence et les injections suite à une chirurgie de la presbytie. Parmi les applications esthétiques, on citera par exemple les injections pour le comblement

des rides, des ridules et des défauts cutanés ou l'augmentation des volumes par exemple ceux des lèvres, des pommettes, etc.

Les applications visées sont plus particulièrement les applications communément répandues dans le cadre des viscoélastiques injectables et des polysaccharides utilisés ou potentiellement utilisables dans les pathologies ou traitements suivants :

- injections esthétiques au niveau du visage : de comblement de rides, défauts cutanés ou volumatrices (pommettes, menton, lèvres) ;
- injections volumatrices au niveau du corps : augmentation des seins et des fesses, augmentation du point G, vaginoplastie, reconstruction des lèvres vaginales, augmentation de la taille du pénis ;
- en chirurgie articulaire et en chirurgie dentaire pour le comblement des poches parodontales par exemple.
- traitement de l'arthrose, injection dans l'articulation en remplacement ou en complément du liquide synovial déficient ;
- injection péri-urétrale pour le traitement de l'incontinence urinaire par insuffisance sphinctérienne ;
- injection post-chirurgicale pour éviter les adhésions péritonéales notamment ;
- injection suite à une chirurgie de la presbytie par incisions sclérales au laser ;
- injection dans la cavité vitréenne ;
- injection au cours de la chirurgie de la cataracte ;
- injection pour le traitement des cas de sécheresses vaginales ;
- injection dans les espaces tissulaires ;
- injection dans les parties génitales.

Plus particulièrement, en chirurgie esthétique, en fonction de ses propriétés viscoélastiques et de rémanence, les formulations obtenues par le procédé objet de l'invention pourront être utilisées :

- pour le comblement des rides fines, moyennes ou profondes, et être injectées avec des aiguilles de diamètre fin (27 Gauge par exemple) ;
- comme volumateur avec une injection par des aiguilles de diamètre plus important, de 22 à 26 Gauge par exemple, et plus longues (30 à 40 mm par exemple); dans ce cas, son caractère cohésif permettra de garantir son maintien à l'emplacement de l'injection.

Ces exemples d'utilisation ne sont nullement limitatifs, et les formulations obtenues selon le procédé objet de l'invention étant plus largement prévue pour :

- combler des volumes ;
- générer des espaces au sein de certains tissus, favorisant ainsi leur fonctionnement optimal ;
- remplacer des liquides physiologiques déficients.

Le composé macromoléculaire selon l'invention n'est pas utilisé en mélange avec un polysaccharide natif dans une formulation. En particulier, le composé macromoléculaire selon l'invention n'est pas utilisé en mélange avec de l'acide hyaluronique ou de l'héparosan dans une formulation.

Dans un mode de réalisation, le composé macromoléculaire selon l'invention est utilisé en mélange avec au moins un polymère réticulé dans une formulation.

Dans un mode de réalisation, le composé macromoléculaire selon l'invention est utilisé en mélange avec au moins un polymère réticulé choisi dans le groupe des polysaccharides réticulés dans une formulation.

Dans un mode de réalisation, le composé macromoléculaire selon l'invention est utilisé en mélange avec au moins un polysaccharide réticulé choisi dans le groupe constitué de l'acide hyaluronique, du kératane, de l'héparine, de la cellulose, des dérivés de cellulose, de l'acide alginique, du xanthane, du carraghénane, du chitosane, de la chondroïtine, de l'héparosan et leurs sels biologiquement acceptables, seuls ou en mélange.

Dans un mode de réalisation, le composé macromoléculaire selon l'invention est utilisé, seul ou en mélange avec au moins un autre polymère, pour la préparation d'un composé macromoléculaire réticulé.

Dans un mode de réalisation, le composé macromoléculaire selon l'invention est utilisé en mélange avec au moins un polysaccharide pour la préparation d'un composé macromoléculaire réticulé.

Dans un mode de réalisation, le polysaccharide utilisé avec le composé macromoléculaire selon l'invention pour la préparation d'un composé macromoléculaire réticulé est choisi dans le groupe constitué de l'acide hyaluronique, du kératane, de l'héparine, de la cellulose, des dérivés de cellulose, de l'acide alginique, du xanthane, du carraghénane, du chitosane, de la chondroïtine, de l'héparosan et leurs sels biologiquement acceptables, seuls ou en mélange, ces polysaccharides étant réticulés ou non réticulés.

Dans un mode de réalisation, le au moins un polysaccharide utilisé avec le composé macromoléculaire selon

l'invention pour la préparation d'un composé macromoléculaire réticulé est choisi dans le groupe constitué de l'acide hyaluronique, de l'héparosan et leurs sels biologiquement acceptables, seuls ou en mélange, ces polysaccharides étant réticulés ou non réticulés.

Dans un mode de réalisation, le polysaccharide utilisé avec le composé macromoléculaire selon l'invention pour la préparation d'un composé macromoléculaire réticulé est un acide hyaluronique ou un sel d'acide hyaluronique.

Dans un mode de réalisation, le polysaccharide utilisé avec le composé macromoléculaire selon l'invention pour la préparation d'un composé macromoléculaire réticulé est un mélange d'acides hyaluroniques ou de sels d'acides hyaluroniques.

Dans un mode de réalisation, le polysaccharide utilisé avec le composé macromoléculaire selon l'invention pour la préparation d'un composé macromoléculaire réticulé est un acide hyaluronique ou un sel d'acide hyaluronique réticulé.

Dans un mode de réalisation, le polysaccharide utilisé avec le composé macromoléculaire selon l'invention pour la préparation d'un composé macromoléculaire réticulé est un héparosan ou un sel d'héparosan.

Dans un mode de réalisation, le polysaccharide utilisé avec le composé macromoléculaire selon l'invention pour la préparation d'un composé macromoléculaire réticulé est un mélange d'héparosan ou de sels d'héparosan.

Dans un mode de réalisation, le polysaccharide utilisé avec le composé macromoléculaire selon l'invention pour la préparation d'un composé macromoléculaire réticulé est un héparosan ou un sel d'héparosan réticulé.

Dans un mode de réalisation, le composé macromoléculaire selon l'invention est utilisé pour la préparation d'au moins un polymère prodrogue.

Dans un mode de réalisation, le composé macromoléculaire selon l'invention est modifié chimiquement par réaction avec au moins un principe actif pour la préparation d'un polymère prodrogue.

Dans un mode de réalisation, le polymère prodrogue obtenu à partir du composé macromoléculaire selon l'invention est utilisé dans le domaine de l'oncologie.

## Exemples

Dans le cadre des exemples, un certain nombre de paramètres ont été mesurés.

Détermination des paramètres rhéologiques G', G" et Tan $\Delta$ : appareillage TA instruments DHR-2. Géométrie type cône avec un angle de 2 ° et un diamètre de 40mm. Méthode d'oscillation en balayage en fréquence (frequency sweep), déformation (strain) de 0,8% sur une plage de fréquence de 0,08 à 5 Hz.

Détermination de la SEC : appareillage Viscotek GPCmax II muni d'un détecteur TDA305 (RI, RALS/LALS, Viscosimètre) et d'une colonne Shodex OHpak SB-806 HQ + SB-805 HQ. Les mesures sont effectuées à 37°C en présence de tampon d'analyse PBS.

## Préparation de compositions selon l'invention

### Exemple 1

Des fibres de hyaluronate de sodium de qualité injectable (10,4 g) de masse moléculaire moyenne en poids de 1 MDa sont pesées dans un récipient. Une solution aqueuse d'hydroxyde de sodium à 1% dans l'eau est ajoutée pour hydrater les fibres de hyaluronate de sodium. Le milieu réactionnel est homogénéisé en alternant agitation mécanique manuelle et repos pendant 50 minutes. 0,43 g de BDDE est ajouté. Le milieu réactionnel comprenant 10,4 g de fibres de hyaluronate de sodium, 55,7 g d'hydroxyde de sodium et 0,43 g de BDDE est de nouveau homogénéisé par agitation mécanique manuelle, puis est placé dans un bain-marie préalablement thermostaté à 2 °C pendant 24 heures.

A l'issue de la réticulation, le milieu réactionnel est neutralisé par ajout d'HCl 1 N et de tampon phosphate sous agitation mécanique. Le gel obtenu est ensuite dialysé contre du tampon phosphate jusqu'à obtenir une concentration en acide hyaluronique de 27,5 mg/g. Après homogénéisation mécanique et débullage, le gel est réparti en seringues verre de 1 mL et traité par stérilisation à la vapeur à 127 °C avec une F0 de 50 minutes. Après ce traitement thermique, les seringues contiennent une solution de composé macromoléculaire. Un échantillon est prélevé à partir d'une seringue, et un test de filtrabilité sur membrane de porosité 0,22 $\mu$m est effectué avec cet échantillon. Le test de filtrabilité est positif : la solution de composé macromoléculaire est filtrable sur membrane de porosité 0,22 $\mu$m.

### Exemple 2

Une composition selon l'invention est préparée par un procédé similaire à celui décrit dans l'exemple 1 mais après ajout du BDDE et homogénéisation, le milieu réactionnel est immédiatement placé dans un bain-marie préalablement thermostaté à 9 °C pendant 3 heures à cette température. Comme décrit dans l'exemple 1, après le traitement thermique, les seringues contiennent une solution de composé macromoléculaire à 27,5 mg/g. Un échantillon est prélevé

à partir d'une seringue, et un test de filtrabilité sur membrane de porosité 0,22 µm est effectué avec cet échantillon. Le test de filtrabilité est positif : la solution de composé macromoléculaire est filtrable sur membrane de porosité 0,22 µm.

**Exemple de référence 3**

Des fibres de hyaluronate de sodium de qualité injectable (10,4 g) de masse moléculaire moyenne en poids de 1 MDa sont pesées dans un récipient. Une solution aqueuse d'hydroxyde de sodium à 1% dans l'eau est ajoutée pour hydrater les fibres de hyaluronate de sodium. Le milieu réactionnel est homogénéisé en alternant agitation mécanique manuelle et repos pendant 50 minutes. Du tampon en remplacement du BDDE est ajouté. Le milieu réactionnel comprenant 2,6 g de fibres de hyaluronate de sodium, 13,9 g d'hydroxyde de sodium et 0,11 g de tampon et de nouveau homogénéisé par agitation mécanique manuelle, puis est placé dans un bain-marie préalablement thermostaté à 9 °C pendant 3 heures.

A l'issue de ces 3 heures d'agitation, le milieu réactionnel est neutralisé par ajout d'HCl 1 N et de tampon phosphate sous agitation mécanique. Le gel obtenu est ensuite dialysé contre du tampon phosphate jusqu'à obtenir une concentration en acide hyaluronique de 27,5 mg/g. Après homogénéisation mécanique et débullage, le gel est réparti en seringues verre de 1 mL et traité par stérilisation à la vapeur à 127 °C avec une F0 de 50 minutes. Après ce traitement thermique, les seringues contiennent une solution de composé macromoléculaire. Un échantillon est prélevé à partir d'une seringue, et un test de filtrabilité sur membrane de porosité 0,22 µm est effectué avec cet échantillon. Le test de filtrabilité est positif : la solution de composé macromoléculaire est filtrable sur membrane de porosité 0,22 µm.

**Propriétés des compositions selon l'invention**

Les propriétés rhéologiques déterminées, des compositions selon l'invention sont données dans le tableau 1 ci-dessous.

**Tableau 1**

| | Exemple 1 | Exemple 2 | Exemple de référence 3 |
|---|---|---|---|
| Polysaccharide | Acide hyaluronique | Acide hyaluronique | Acide hyaluronique |
| G' (Pa) à 1Hz de la solution | 86 | 68 | 44 |
| Tan Δ à 1Hz de la solution | 1,00 | 1,16 | 2,07 |
| Filtrabilité sur membrane de porosité 0,22 µm à 20 mg/g | OUI | OUI | OUI |

Les compositions selon l'invention sont filtrables sur membrane de porosité 0,22 µm et ont une Tan Δ supérieure ou égal à 1.

**Comportements hydrodynamiques des compositions selon l'invention en comparaison de la composition de référence 3**

Les compositions ont été soumises à une analyse chromatographique par exclusion stérique dans le but de déterminer les coefficients de la relation de Mark-Houwink qui s'écrit :

$$[\eta] = K.M^a$$

[η] étant la viscosité intrinsèque.
M étant la masse molaire moyenne viscosimétrique.

Cette relation linéaire permet de déterminer aisément les valeurs des coefficients a et K. Si l'on représente log([η]) en fonction de log(M), on obtient une droite de pente a et d'ordonnée à l'origine log(K).

En figure 1, pour chacune des compositions est tracée la courbe log(viscosité intrinsèque-dL/g) en fonction de log(masse moléculaire-Da) et la corrélation linéaire est calculée.

Les valeurs mesurées et calculées sont rassemblées dans le tableau 2 ci-dessous.

**Tableau 2**

| Gel testé | Courbes | Equation de la régression linéaire | Coefficient de corrélation | Coefficient « a » de la relation de Mark Houwink | [η] Viscosité intrinsèque pour Masse moléculaire = 1 000 kDa |
|---|---|---|---|---|---|
| Exemple 1 | o | y = 0,564x - 2,238 | $R^2$ = 0,9941 | a = 0,56 | 14,1 dL/g |
| Exemple 2 | + | y = 0,567x - 2,244 | $R^2$ = 0,9947 | a = 0,57 | 14,5 dL/g |
| Exemple de référence 3 | □ | y = 0,615x - 2,445 | $R^2$ = 0,9938 | a = 0,62 | 17,8 dL/g |

On observe que les acides hyaluroniques des compositions selon l'invention ont une viscosité intrinsèque et un coefficient de Mark Houwink similaires à ceux de l'acide hyaluronique de la composition de l'exemple de référence 3 et ne sont donc plus réticulés.

Néanmoins ils présentent un G' et une Tan Δ très différents, adaptés aux applications ci-dessus citées.

## Revendications

1. Composition, sous forme de solution aqueuse, filtrable sur membrane de porosité 0,22 µm comprenant au moins un composé macromoléculaire constitué d'enchaînements de polysaccharides identiques ou différents liés entre eux par un radical divalent L.

2. Composition selon la revendication 1, **caractérisée en ce que** le composé macromoléculaire a pour formule générale I :

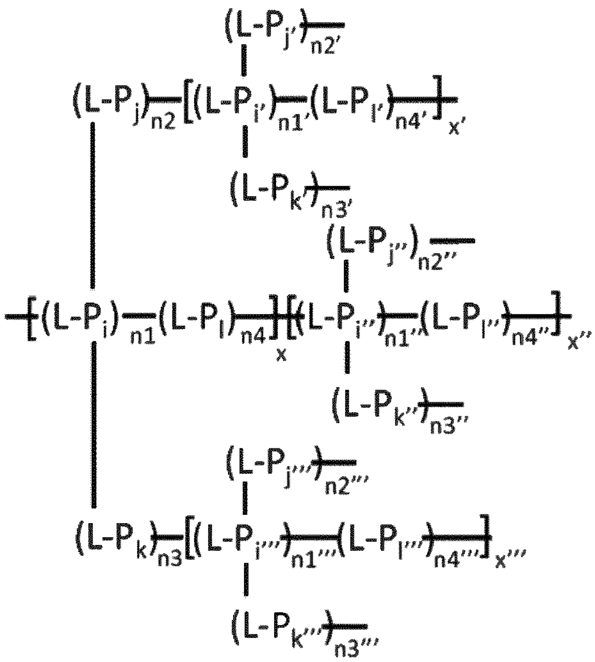

Formule I

dans laquelle :

- $P_i$, $P_{i'}$, $P_{i''}$, $P_{i'''}$, $P_j$, $P_{j'}$, $P_{j''}$, $P_{i'''}$, $P_k$, $P_{k'}$, $P_{k''}$, $P_{k'''}$, $P_l$, $P_{l'}$, $P_{l''}$ et $P_{l'''}$ sont des polysaccharides identiques ou différents,
- n1, n1', n1", n1‴, n2, n2', n2", n2‴, n3, n3', n3", n3‴, n4, n4', n4" et n4‴ sont des entiers compris entre 0 et

2000 et au moins un des ni, n1', n1", n1‴, n2, n2', n2", n2‴, n3, n3', n3", n3‴, n4, n4', n4" et n4‴ est supérieur ou égal à 2,
- x, x', x" et x'" sont des entiers supérieurs ou égaux à 0,
- L est un radical divalent issu d'une réaction entre un agent réticulant et deux fonctions réactives, chaque fonction réactive étant portée par deux chaines distinctes de polysaccharide,
- les enchaînements de polysaccharides ne forment pas de structures cycliques.

**3.** Composition selon la revendication 1, **caractérisée en ce que** le composé macromoléculaire est constitué d'un enchaînement de polysaccharides choisis dans le groupe constitué de l'acide hyaluronique, du kératane, de l'héparine, de la cellulose, des dérivés de cellulose, de l'acide alginique, du xanthane, du carraghénane, du chitosan, de la chondroïtine, de l'héparosan, et leurs sels biologiquement acceptables, seul(s) ou en mélange.

**4.** Composé macromoléculaire de formule générale I :

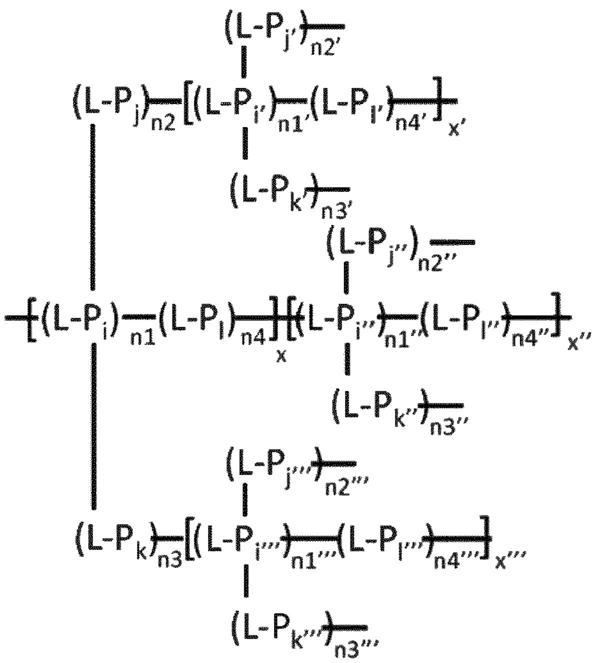

Formule I

dans laquelle :

- $P_i$, Pi', $P_{i''}$, $P_{i'''}$, $P_j$, $P_{j'}$, $P_{j''}$, $P_{i'''}$, Pk, $P_{k'}$, $P_{k''}$, $P_{k'''}$, $P_l$, Pi', $P_{l''}$ et $P_{l'''}$ sont des polysaccharides identiques ou différents,
- ni, n1', n1", n1‴, n2, n2', n2", n2‴, n3, n3', n3", n3‴, n4, n4', n4" et n4‴ sont des entiers compris entre 0 et 2000 et au moins un des ni, n1', n1", n1‴, n2, n2', n2", n2‴, n3, n3', n3", n3‴, n4, n4', n4" et n4‴ est supérieur ou égal à 2,
- x, x', x" et x'" sont des entiers supérieurs ou égaux à 0,
- L est un radical divalent issu d'une réaction entre un agent réticulant et deux fonctions réactives, chaque fonction réactive étant portée par deux chaines distinctes de polysaccharide,
- les enchaînements de polysaccharides ne forment pas de structures cycliques.

**5.** Procédé de préparation d'une composition selon la revendication 1, comprenant au moins les étapes suivantes :

a) on dispose d'au moins un polysaccharide
b) on dispose d'au moins un agent réticulant
c) on met en œuvre une ou plusieurs étape(s) de réticulation en présence dudit polysaccharide et dudit agent réticulant
d) on obtient un polysaccharide réticulé
e) on met en œuvre une ou plusieurs étape(s) de rupture de liaisons glycosidiques
f) on obtient une solution de composé macromoléculaire
g) on filtre la solution de composé macromoléculaire sur membrane de porosité 0,22 µm.

6. Procédé selon la revendication 5, **caractérisé en ce que** l'agent réticulant est choisi dans le groupe constitué par les bis-époxydes, les trimétaphosphates, les diamines, les dialcoxyamines et les dihydrazides.

7. Procédé selon l'une quelconque des revendications 5 et 6, **caractérisé en ce que** l'étape e) de rupture de liaisons glycosidiques est effectuée au moyen d'un traitement chimique.

8. Procédé selon l'une quelconque des revendications 5 et 6,**caractérisé en ce que** l'étape e) de rupture de liaisons glycosidiques est effectuée au moyen d'un traitement thermique.

9. Procédé selon l'une quelconque des revendications 5 et 6,**caractérisé en ce que** l'étape e) de rupture de liaisons glycosidiques est effectuée au moyen d'un traitement par radiations.

10. Procédé selon l'une quelconque des revendications 5 et 6,**caractérisé en ce que** l'étape e) de rupture de liaisons glycosidiques est effectuée au moyen d'un traitement enzymatique.

11. Procédé selon l'une quelconque des revendications 5 et 6,**caractérisé en ce que** l'étape e) de rupture de liaisons glycosidiques est effectuée au moyen d'un traitement à haute pression.

12. Composition selon l'une quelconque des revendications 1 à 3,**caractérisée en ce que** Tan $\Delta$ (Tn $\delta$) $\geq$ 1 à 1 Hz.

13. Formulation comprenant au moins une composition selon l'une quelconque des revendications 1 à 3.Formulation selon la revendication 13,, **caractérisée en ce qu'**elle comprend en outre au moins un actif.

14. Formulation selon la revendication 13, **caractérisée en ce que** l'actif est choisi dans le groupe constitué des anesthésiques locaux, des antioxydants, des antiinflammatoires, des vitamines, des acides aminés, des vasoconstricteurs, des vasodilatateurs, des anti-hémorragiques ou hémostatiques, des antimicrobiens, des glycosides et leurs dérivés, des hydratants ou régénérants tissulaires, seul(s) ou en mélange.

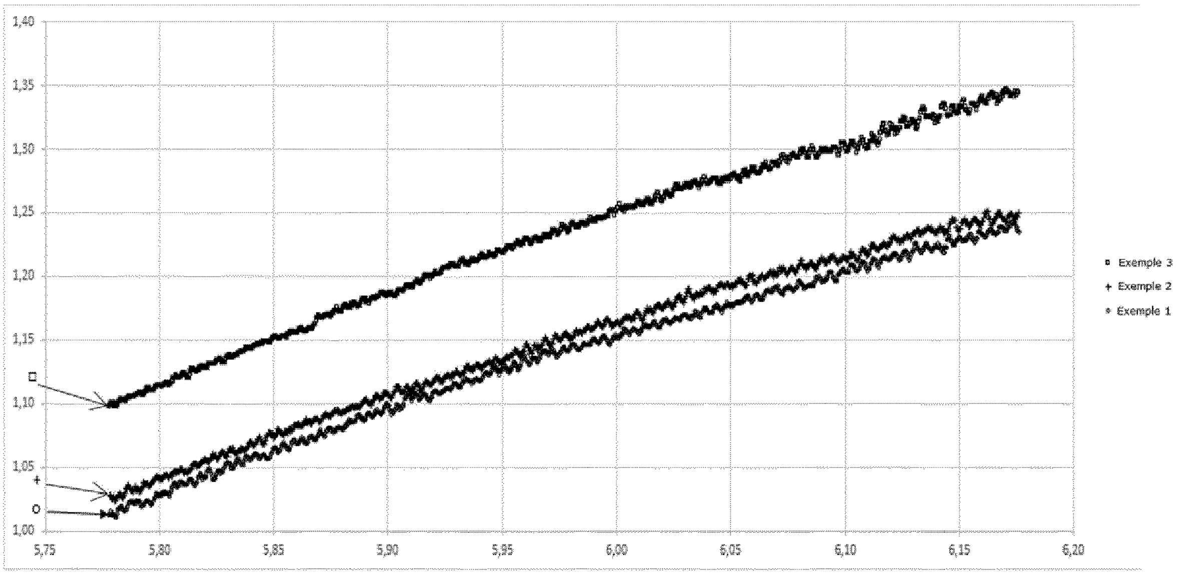
1,40
1,35
1,30
1,25
1,20
1,15
1,10
1,05
1,00
5,75
5,80
5,85
5,90
5,95
6,00
6,05
6,10
6,15
6,20
Exemple 3
Exemple 2
Exemple 1

Figure 1

Europäisches Patentamt
European Patent Office
Office européen des brevets

# RAPPORT DE RECHERCHE EUROPEENNE

Numéro de la demande
EP 21 20 6821

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2020/250128 A1 (OFFHEALTH S P A [IT]) 17 décembre 2020 (2020-12-17) * page 1, lignes 4-8; revendications 1, 3 * ----- | 1-14 | INV. C08B37/08 C08L5/08 A61K31/728 A61K47/36 |
| X | WO 2016/030516 A1 (GALDERMA SA [CH]) 3 mars 2016 (2016-03-03) * exemple 5 * ----- | 1-14 | |
| | | | DOMAINES TECHNIQUES RECHERCHES (IPC) |
| | | | C08B C08L A61K |

Le présent rapport a été établi pour toutes les revendications

1

| Lieu de la recherche | Date d'achèvement de la recherche | Examinateur |
|---|---|---|
| Munich | 6 avril 2022 | Pellegrini, Paolo |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 21 20 6821

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

06-04-2022

| Document brevet cité au rapport de recherche | Date de publication | Membre(s) de la famille de brevet(s) | Date de publication |
|---|---|---|---|
| WO 2020250128 A1 | 17-12-2020 | EP 3962495 A1 | 09-03-2022 |
| | | WO 2020250128 A1 | 17-12-2020 |
| WO 2016030516 A1 | 03-03-2016 | EP 3194452 A1 | 26-07-2017 |
| | | US 2017291963 A1 | 12-10-2017 |
| | | WO 2016030516 A1 | 03-03-2016 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2983483


### Littérature non-brevet citée dans la description


- **L. NORD ; A. EMILSON ; C. STURESSON ; A.H. KENNE.** Degree of Modification of Hyaluronic Acid Dermal Fillers. *18th Congress of the EADV, Berlin,* 2009